(19) Europäisches Patentamt
European Patent Office
Office européen des brevets

(11) Veröffentlichungsnummer: **0 378 508 B1**

(12) **EUROPÄISCHE PATENTSCHRIFT**

(45) Veröffentlichungstag der Patentschrift: **01.03.95**

(51) Int. Cl.[6]: **C07D 487/04**, A01N 25/32, //(C07D487/04,249:00,239:00)

(21) Anmeldenummer: **90810001.9**

(22) Anmeldetag: **04.01.90**

# (54) Antidots zur Verbesserung der Kulturpflanzenverträglichkeit agrochemischer Wirkstoffe.

(30) Priorität: **11.01.89 CH 78/89**
**06.11.89 CH 3987/89**

(43) Veröffentlichungstag der Anmeldung:
**18.07.90 Patentblatt 90/29**

(45) Bekanntmachung des Hinweises auf die Patenterteilung:
**01.03.95 Patentblatt 95/09**

(84) Benannte Vertragsstaaten:
**AT BE CH DE ES FR GR IT LI LU**

(56) Entgegenhaltungen:
**EP-A- 0 112 289**
**DE-A- 2 128 498**
**GB-A- 2 149 792**

(73) Patentinhaber: **CIBA-GEIGY AG**
**Klybeckstrasse 141**
**CH-4002 Basel (CH)**

(72) Erfinder: **Seiler, Alfred, Dr.**
**Schaffhauserstrasse 51**
**CH-4332 Stein (CH)**
Erfinder: **Schneider, Hans-Dieter, Dr.**
**Efringerstrasse 32**
**D-7858 Weil am Rhein (DE)**
Erfinder: **Dürr, Dieter, Dr.**
**Brändelistalweg 16**
**CA-4103 Bottmingen (CH)**

**EP 0 378 508 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die neue Verwendung von Antidots der Formel I zum Schützen von Kulturpflanzen gegen die phytotoxische Wirkung von Herbiziden. Ferner betrifft die Erfindung neue Verbindungen und ihre Herstellung sowie neue Zwischenprodukte und ihre Herstellung. Weiter betrifft die Erfindung herbizide Mittel, die eine Kombination von Herbizid und Antidot enthalten sowie Verfahren zur selektiven Bekämpfung von Unkräutern mittels Herbizid und Antidot. Schliesslich betrifft die Erfindung auch das durch die Behandlung mit dem Antidot geschützte Saatgut von Kulturpflanzen.

Es ist bekannt, dass Herbizide aus den Stoffklassen der Sulfonylharnstoffe, der Halogenacetanilide, der Aryloxyphenoxypropionsäurederivate und der N-Benzoyl-N-phenylalanine bei der Anwendung in wirksamer Dosis gelegentlich neben den zu bekämpfenden Unkräutern auch die Kulturpflanzen in gewissem Masse schädigen. Ueberdosen werden oft ungewollt und zufälligerweise appliziert, wenn sich Randzonen beim streifenweisen Spritzen überdecken, sei es durch Windeinwirkung oder durch falsches Einschätzen der Breitenwirkung des Spritzgerätes. Zu einer Schädigung von Kulturpflanzen kann es auch im Rahmen der Fruchtfolge kommen, wenn nach Kulturpflanzen, welche gegen das eingesetzte Herbizid resistent sind, andere Kulturpflanzen angebaut werden, welche gegenüber besagtem Herbizid nicht oder nur unzureichend resistent sind. Es können klimatische Verhältnisse oder eine Bodenbeschaffenheit vorliegen, bei denen die für normale Bedingungen empfohlene Herbizidmenge als Ueberdosis wirkt. Die Qualität des Saatgutes kann bei der Herbizidverträglichkeit auch eine Rolle spielen. Um dem Problem unzureichender Selektivität von Herbiziden zu begegnen, sind schon verschiedene Stoffe vorgeschlagen worden, welche befähigt sind, die schädigende Wirkung des Herbizids auf die Kulturpflanze spezifisch zu antagonisieren, das heisst die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, das heisst, ein bestimmtes Gegenmittel eignet sich oft nur für eine bestimmte Kulturpflanze und einige wenige herbizide Stoffklassen.

So beschreibt die Britische Patentschrift 1,277,557 die Behandlung von Samen oder Sprösslingen von Weizen und Sorghum mit gewissen Oxamsäureestern und Amiden zum Schutz vor dem Angriff durch "ALACHLOR" (N-Methoxymethyl-N-chloracetyl-2,6-diäthylanilin). Im US-Patent 4,618,331 werden Benzoxazinderivate mit Schutzwirkung vor der herbiziden Wirkung von Halogenacetaniliden und Sulfonylharnstoffen offenbart. Zum Schutz gegen Sulfonylharnstoffherbizide werden als Gegenmittel in der EP-A-122 231 Benzoyloximäther und in der EP-A-147 365 Phenylglyoxylsäurenitril-oxim, Naphthalindicarbonsäureanhydrid, ein Thiazolcarbonsäureester sowie Dichloracetamide vorgeschlagen. Ferner können Maispflanzen gemäss der DE-OS 2,402,983 wirksam vor Schädigung durch Chloracetanilide geschützt werden, indem man dem Boden als Gegenmittel ein N-disubstituiertes Dichloracetamid zuführt. Derartige Verbindungen werden gemäss DE-OS 2,828,265 und 2,828,293 auch als Antidots gegen herbizide Acetanilide verwendet.

Der Einsatz von Thiophencarbonsäureestern als Antidots zum Schützen von Kulturpflanzen gegen Sulfonylharnstoff-Herbizide wird in der EP-A-127 469 beschrieben.

Es wurde nun gefunden, dass sich überraschenderweise Verbindungen der Formel I

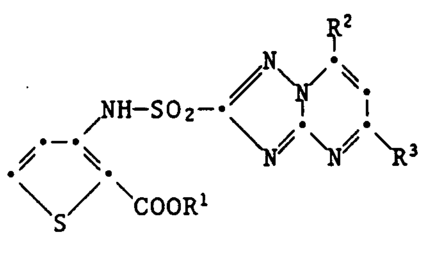

(I),

worin

| | |
|---|---|
| $R^1$ | $C_1$-$C_6$-Alkyl oder $C_3$-$C_6$-Cycloalkyl und |
| $R^2$ und $R^3$ | unabhängig voneinander Wasserstoff, $C_1$-$C_3$-Alkyl, Trifluormethyl oder Cyclopropyl bedeuten, |

mit der Massgabe, dass nur eines der Symbole $R^2$ und $R^3$ für Wasserstoff stehen kann,
hervorragend dazu eignen, Kulturpflanzen gegen die schädigende Wirkung von Herbiziden zu schützen. Die Verbindungen der Formel I werden im folgenden auch als "Gegenmittel", "Antidots" oder "Safener" bezeichnet.

Besonders geeignet zum Schützen von Kulturpflanzen sind

a) Antidots der Formel I, in welchen $R^2$ und $R^3$ unabhängig voneinander $C_1$-$C_3$-Alkyl oder Cyclopropyl bedeuten und $R^1$ die für Formel I angegebenen Bedeutungen hat;

b) Antidots der Formel I, in welchen eines der Symbole $R^2$ und $R^3$ für Methyl und das andere für Cyclopropyl steht und $R^1$ die für Formel I angegebenen Bedeutungen hat;

c) Antidots der Formel I, in welchen $R^1$ Methyl bedeutet und $R^2$ und $R^3$ die für Formel I angegebenen Bedeutungen haben,

d) Antidots der Formel I, in welchen $R^1$ $C_1$-$C_3$-Alkyl und $R^2$ und $R^3$ unabhängig voneinander Wasserstoff, Methyl oder Cyclopropyl bedeuten, mit der Massgabe, dass nur eines der Symbole $R^2$ und $R^3$ für Wasserstoff stehen kann, und insbesondere das Antidot [N-(5,7-Dimethyl-1,2,4-triazolo[1,5-a]pyrimidin-2-ylsulfonyl)-3-aminothienyl-2]-carbonsäuremethylester der Formel Ia

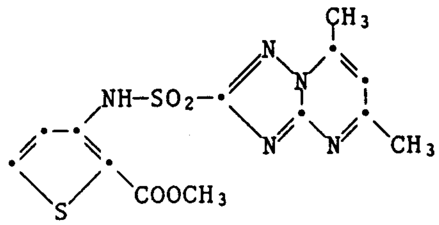

(Ia).

Die Verbindung der Formel Ia ist aus GB 2 149 792 bekannt.

Die Erfindung betrifft ferner neue, unter die Formel I fallende Verbindungen der Formel I'

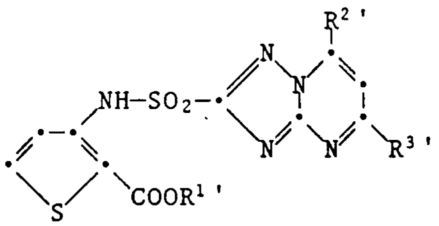

(I'),

worin

| | |
|---|---|
| $R^{1'}$ | $C_1$-$C_6$-Alkyl oder $C_3$-$C_6$-Cycloalkyl und |
| $R^{2'}$ und $R^{3'}$ | unabhängig voneinander Wasserstoff, $C_1$-$C_3$-Alkyl, Trifluormethyl oder Cyclopropyl bedeuten, |

mit der Massgabe, dass mindestens eines der Symbole $R^{2'}$ und $R^{3'}$ für Cyclopropyl steht, sowie die Herstellung dieser Verbindungen und dafür verwendete Zwischenprodukte.

Von den Verbindungen der Formel I' sind besonders diejenigen erwähnenswert, in denen

a) eines der Symbole $R^{2'}$ und $R^{3'}$ für Cyclopropyl und das andere für Methyl steht und $R^{1'}$ die für Formel I' angegebenen Bedeutungen hat;

b) $R^{1'}$ für Methyl steht und $R^{2'}$ und $R^{3'}$ die für Formel I angegebenen Bedeutungen haben; und

c) $R^{1'}$ für Methyl steht und eines der Symbole $R^{2'}$ und $R^{3'}$ für Cyclopropyl und das andere für Methyl steht.

Besonders hervorzuheben ist der N-(7-Cyclopropyl-5-methyl-1,2,4-triazolo(1,5-a]pyrimidin-2-ylsulfonyl)-3-aminothiophen-2-carbonsäuremethylester.

Ein Gegenmittel oder Antidot der Formel I kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidot kann daher grundsätzlich unabhängig vom Zeitpunkt der Applikation des Herbizids erfolgen. Die Behandlung der Pflanze kann jedoch auch durch gleichzeitige Applikation von Herbizid und Gegenmittel (Tankmischung) erfolgen. Die pre-emergente Behandlung schliesst sowohl die Behandlung der Anbaufläche vor der Aussaat (ppi = pre plant incorporation) als auch die Behandlung der angesäten, aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1:100 bis 100:1. In der Regel wird bei einem Mengenverhältnis von Gegenmittel zu Herbizid von 10:1 bis 1:10 die volle Schutzwirkung erreicht. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid benötigt. Im allgemeinen werden bei der Samenbeizung pro kg Samen 0,01-6,0 g Gegenmittel benötigt, wobei jedoch meistens mit 0,1-2,0 g Gegenmittel pro kg Samen bereits die volle Schutzwirkung erreicht wird. Falls das Gegenmittel kurz vor der Aussaat durch Samenquellung (seed soaking) appliziert werden soll, so werden zweckmässigerweise Lösungen des Gegenmittels verwendet, welche den Wirkstoff in einer Konzentration von 1-10000 ppm enthalten. In der Regel wird mit Konzentrationen des Gegenmittels von 100-1000 ppm die volle Schutzwirkung erreicht.

Häufig liegt zwischen protektiven Massnahmen, insbesondere bei der Samenbeizung und bei der Behandlung von Stecklingen mit einem Gegenmittel der Formel I und der möglichen späteren Feldbehandlung mit Herbiziden ein längerer Zeitraum. Die Erfindung bezieht sich daher auch auf für Kulturpflanzen protektive Mittel, welche aus einem Gegenmittel der Formel I' und ein oder mehreren Hilfsstoffen, Trägerstoffen oder Hilfs- und Trägerstoffen bestehen. Ferner betrifft die Erfindung herbizide Mittel, welche als Gegenmittel eine Verbindung der Formel I und ein Herbizid, vor dessen Einfluss die Kulturpflanze geschützt werden soll, enthalten, gegebenenfalls zusammen mit Hilfsstoffen, Trägerstoffen oder Hilfs- und Trägerstoffen. Ebenfalls Gegenstand der Erfindung ist das mit einem Wirkstoff der Formel I vorbehandelte Vermehrungsgut von Kulturpflanzen wie Saatgut, Setzlinge oder Stecklinge. Insbesondere sind die Wirkstoffe der Formel I zur Behandlung des Saatguts von Soja und Getreide geeignet. Im Rahmen der vorliegenden Erfindung umfasst der Begriff Getreide sämtliche Getreidearten. Hierzu gehören vor allem Weizen, Roggen, Gerste, Hafer, Reis, Sorghum und Mais.

Als Kulturpflanzen gelten im Rahmen vorliegender Erfindung Pflanzen, welche Ertragsstoffe produzieren und zu diesem Zweck ausgebaut werden, insbesondere solche Pflanzen, welche der menschlichen Ernährung dienen, wie vor allem Soja und Getreide.

Hervorragende Schutzwirkung gegen Sulfonylharnstoffherbizide, gegen N-Benzoyl-N-phenylalaninherbizide, gegen Aryloxyphenoxypropionsäureherbizide und gegen Chloracetanilidherbizide werden bei Anwendung von Antidots der Formel I vor allem in Weizen, Roggen, Gerste, Hafer und Reis beobachtet.

Sulfonylharnstoffherbizide, deren schädigende Wirkung gegenüber Kulturpflanzen durch Verbindungen der Formel I aufgehoben oder herabgesetzt werden kann, sind in letzter Zeit in grosser Zahl bekannt geworden. Aus der Vielzahl der Publikationen, welche herbizid wirksame Sulfonylharnstoffderivate offenbaren, sollen beispielhaft das US-Patent 4 127 405 sowie die publizierten Europäischen Patentanmeldungen EP-A-7687, EP-A-30142, EP-A-44807, EP-A-44808, EP-A-51466, EP-A-70802, EP-A-84020, EP-A-87780, EP-A-102925, EP-A-108708, EP-A-120814, EP-A-136061, EP-A-184385, EP-A-206995 und EP-A-237292 genannt sein.

Typische Vertreter von herbiziden Sulfonylharnstoffderivaten, deren schädigende Wirkung auf Kulturpflanzen durch Antidots der Formel I aufgehoben oder herabgesetzt werden kann, sind unter der Formel II

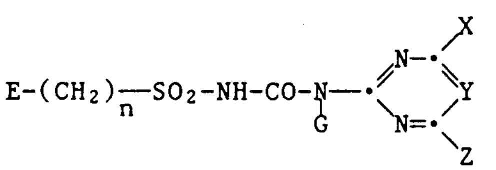

(II)

zusammengefasst, worin

E eines der Strukturelemente

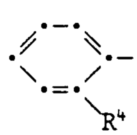 , 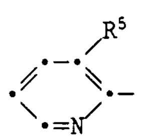 , 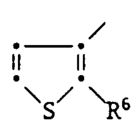 oder 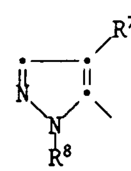 ,

n die Zahl null oder eins,

G Wasserstoff oder Methyl,

X Methoxy, Aethoxy, Difluormethoxy, Methyl oder Chlor
Y CH oder N,
Z Methoxy, Methyl, Difluormethoxy, Cyclopropyl oder Methylamino,
$R^4$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R^5$ Trifluormethyl oder Di($C_1$-$C_4$-alkyl)carbamoyl,
$R^6$ $C_1$-$C_4$-Alkoxycarbonyl,
$R^7$ $C_1$-$C_4$-Alkoxycarbonyl, und
$R^8$ $C_1$-$C_4$-Alkyl bedeuten.

Unter die Formel II fallen folgende herbizide Einzelwirkstoffe:
N-(3-Trifluormethylpyridin-2-ylsulfonyl)-N'-(4,6-dimethoxypyrimidin-2-yl)-harnstoff,
N-(3-Dimethylcarbamoylpyridin-2-ylsulfonyl)-N'-(4,6-dimethoxypyrimidin-2-yl)-harnstoff,
N-(1-Methyl-4-ethoxycarbonylpyrazol-5-ylsulfonyl)-N'-(4,6-dimethoxypyrimidin-2-yl)-harnstoff,
N-(2-Methoxycarbonylthien-3-ylsulfonyl)-N'-(4-methoxy-6-methyl-1,3,5-triazin-2-yl)-harnstoff,
N-(2-Methoxycarbonylbenzylsulfonyl)-N'-(4,6-dimethoxypyrimidin-2-yl)-harnstoff,
N-(2-Methoxycarbonylphenylsulfonyl)-N'-(4,6-bis-difluormethoxypyrimidin-2-yl)-harnstoff,
N-(2-Methoxycarbonylphenylsulfonyl)-N'-(4-ethoxy-6-methylamino-1,3,5-triazin-2-yl)-harnstoff,
N-(2-Methoxycarbonylphenylsulfonyl)-N'-(4-methoxy-6-methyl-1,3,5-triazin-2-yl)-harnstoff,
N-(2-Ethoxycarbonylphenylsulfonyl)-N'-(4-chlor-6-methoxypyrimidin-2-yl)-harnstoff,
N-(2-Methoxycarbonylphenylsulfonyl)-N'-(4-methoxy-6-methyl-1,3,5-triazin-2-yl)-N'-methylharnstoff,
N-(2-Methoxycarbonylphenylsulfonyl)-N'-(4,6-dimethoxypyrimidin-2-yl)-harnstoff,
N-(2-Chlorphenylsulfonyl)-N'-(4-methoxy-6-methyl-1,3,5-triazin-2-yl)-harnstoff,
N-[2-(2-Chlorethoxy)-phenylsulfonyl]-N'-(4-methoxy-6-methyl-1,3,5-triazin-2-yl)-harnstoff, und besonders hervorzuheben ist N-[2-(2-Methoxyethoxy)-phenylsulfonyl]-N'-(4,6-dimethoxy-1,3,5-triazin-2-yl)-harnstoff.

Chloracetanilide, deren schädigende Wirkung gegenüber Kulturpflanzen durch Verbindungen der Formel I aufgehoben werden kann, sind ebenfalls bereits in grosser Zahl bekannt geworden. Solche Chloracetanilide können durch die folgende allgemeine Formel III beschrieben werden:

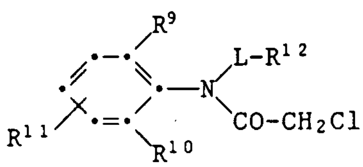

(III)

worin
L eine $C_1$-$C_5$-Alkylenbrücke, insbesondere eine $C_1$-$C_4$-Alkylenbrücke, $R^9$, $R^{10}$ und $R^{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R^{12}$ Wasserstoff, $C_1$-$C_4$-Alkoxy, -COOH, $C_1$-$C_4$-Alkoxycarbonyl, $-CONH_2$, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes 1,2,4-Triazol-3-yl,
gegebenenfalls substituiertes Dioxolanyl,
gegebenenfalls substituiertes Dioxanyl oder
gegebenenfalls substituiertes Tetrahydrofuryl, bedeutet, oder das Strukturelement $-L-R^{12}$ für eine durch zwei $C_1$-$C_3$-Alkoxygruppen substituierte $C_1$-$C_4$-Alkylenbrücke oder für 5-Methyl-1,3,4-oxadiazol-2-yl steht.

Die Alkylenbrücke L kann geradkettig oder verzweigt sein.

Bevorzugt sind Chloracetanilide der Formel III, worin L eine $C_1$-$C_4$-Alkylenbrücke, $R^9$, $R^{10}$ und $R^{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und $R^{12}$ $C_1$-$C_4$-Alkoxy, -COOH, $C_1$-$C_4$-Alkoxycarbonyl, $-CONH_2$, $C_1$-

$C_4$-Alkylcarbamoyl, Di-$C_1$-$C_4$-alkylcarbamoyl, Cyan oder $C_1$-$C_4$-Alkylcarbonyl bedeutet.

Unter die Formel III fallen insbesondere die folgenden herbiziden Chloracetanilidderivate:

N-Ethoxymethyl-N-chloracetyl-2-ethyl-6-methylanilin,
N-Chloracetyl-N-methoxymethyl-2,6-diethylanilin,
N-Chloracetyl-N-(2-methoxyethyl)-2,6-dimethylanilin,
N-Chloracetyl-N-(2-n-propoxyethyl)-2,6-dimethylanilin,
N-Chloracetyl-N-(2-isopropoxyethyl)-2,6-dimethylanilin,
N-Chloracetyl-N-(2-methoxyethyl)-2-ethyl-6-methylanilin,
N-Chloracetyl-N-(2-methoxyethyl)-2,6-diethylanilin,
N-(2-Ethoxyethyl)-N-chloracetyl-2-ethyl-6-methylanilin,
N-Chloracetyl-N-(2-methoxy-1-methylethyl)-2-methylanilin,
N-Chloracetyl-N-(2-methoxy-1-methylethyl)-2,6-dimethylanilin,
N-Chloracetyl-N-(2-methoxy-1-methylethyl)-2,6-diethylanilin,
N-Chloracetyl-N-(2-methoxy-1-methylethyl)-2-ethyl-6-methylanilin,
N-(2-Ethoxyethyl)-N-chloracetyl-2,6-diethylanilin,
N-Chloracetyl-N-(2-n-propoxyethyl)-2-ethyl-6-methylanilin,
N-Chloracetyl-N-(2-n-propoxyethyl)-2,6-diethylanilin,
N-Chloracetyl-N-(2-isopropoxyethyl)-2-ethyl-6-methylanilin,
N-Ethoxycarbonylmethyl-N-chloracetyl-2,6-dimethylanilin
N-Ethoxycarbonylmethyl-N-chloracetyl-2,6-diethylanilin
N-Chloracetyl-N-methoxycarbonylmethyl-2,6-dimethylanilin,
N-Chloracetyl-N-(2,2-diethoxyethyl)-2,6-dimethylanilin,
N-Chloracetyl-N-(2-methoxy-1-methylethyl)-2,3-dimethylanilin,
N-(2-Ethoxyethyl)-N-chloracetyl-2-methylanilin,
N-Chloracetyl-N-(2-methoxyethyl)-2-methylanilin,
N-Chloracetyl-N-(2-methoxy-2-methylethyl)-2,6-dimethylanilin,
N-(2-Ethoxy-2-methylethyl)-N-chloracetyl-2-ethyl-6-methylanilin,
N-Chloracetyl-N-(1-ethyl-1-methoxyethyl)-2,6-dimethylanilin,
N-Chloracetyl-N-(2-methoxyethyl)-2-methoxy-6-methylanilin,
N-n-Butoxymethyl-N-chloracetyl-2-tert.-butylanilin,
N-(2-Ethoxyethyl-2-methylethyl)-N-chloracetyl-2,6-dimethylanilin,
N-Chloracetyl-N-(-2-methoxyethyl)-2-chlor-6-methylanilin,
N-(2-Ethoxyethyl)-N-chloracetyl-2-chlor-6-methylanilin,
N-(2-Ethoxyethyl)-N-chloracetyl-2,3,6-trimethylanilin,
N-Chloracetyl-N-(2-methoxyethyl)-2,3,6-trimethylanilin,
N-Chloracetyl-N-cyanomethyl-2,6-dimethylanilin,
N-Chloracetyl-N-(1,3-dioxolan-2-ylmethyl)-2,6-dimethylanilin,
N-Chloracetyl-N-(1,3-dioxolan-2-ylmethyl)-2-ethyl-6-methylanilin,
N-Chloracetyl-N-(1,3-dioxan-2-ylmethyl)-2-ethyl-6-methylanilin,
N-Chloracetyl-N-(2-furylmethyl)-2,6-dimethylanilin,
N-Chloracetyl-N-(2-furylmethyl)-2-ethyl-6-methylanilin,
N-Chloracetyl-N-(2-tetrahydrofurylmethyl)-2,6-dimethylanilin,
N-Chloracetyl-N-(N,N-dimethylcarbamoylmethyl)-2,6-dimethylanilin,
N-(n-Butoxymethyl)-N-chloracetyl-2,6-diethylanilin,
N-(2-n-Butoxyethyl)-N-chloracetyl-2,6-diethylanilin,
N-Chloracetyl-N-(2-methoxy-1,2-dimethylethyl)-2,6-dimethylanilin,
N-Chloracetyl-N-isopropyl-2,3-dimethylanilin,
N-Chloracetyl-N-isopropyl-2-chloranilin,
N-Chloracetyl-N-(1H-pyrazol-1-ylmethyl)-2,6-dimethylanilin,
N-Chloracetyl-N-(1H-pyrazol-1-ylmethyl)-2-ethyl-6-methylanilin,
N-Chloracetyl-N-(1H-1,2,4-triazol-1-ylmethyl)-2,6-dimethylanilin,
N-Chloracetyl-N-(1H-1,2,4-triazol-1-ylmethyl)-2,6-diethylanilin,
N-Benzoylmethyl-N-chloracetyl-2,6-diethylanilin,
N-Benzoylmethyl-N-chloracetyl-2-ethyl-6-methylanilin,
N-Chloracetyl-N-(5-methyl-1,3,4-oxadiazol-2-yl)-2,6-diethylanilin,
N-Chloracetyl-N-(5-methyl-1,3,4-oxadiazol-2-yl)-2-ethyl-6-methylanilin,
N-Chloracetyl-N-(5-methyl-1,3,4-oxadiazol-2-yl)-2-tert.butylanilin,
N-Chloracetyl-N-(4-chlorbenzoylmethyl)-2,6-dimethylanilin und

N-Chloracetyl-N-(4-methyl-5-methylthio-1,2,4-triazol-3-ylmethyl)-2,6-diethylanilin.

Aryloxyphenoxypropionsäureherbizide, deren schädigende Wirkung gegenüber Kulturpflanzen durch Antidots der Formel I aufgehoben oder herabgesetzt werden kann, sind in grosser Zahl bekannt. Solche Aryloxyphenoxypropionsäurederivatekönnen durch die folgende allgemeine Formel IV beschrieben werden:

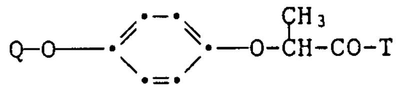

(IV)

worin
Q für den Rest

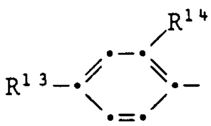 ,

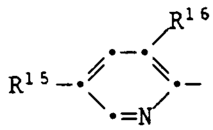 ,

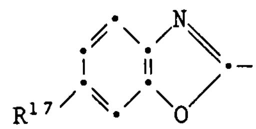 ,

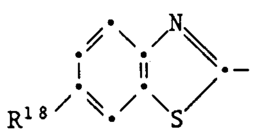

oder

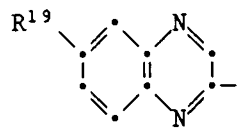

und T für
$-NR^{20}R^{21}$, $-N(CN)R^{22}$, $-OR^{23}$ $SR^{24}$ oder $-O-N=CR^{25}R^{26}$ stehen, wobei

| | |
|---|---|
| $R^{13}$ und $R^{15}$ | Halogen oder Trifluormethyl, |
| $R^{14}$, $R^{16}$, $R^{17}$, $R^{18}$ und $R^{19}$ | Wasserstoff oder Halogen, |
| $R^{20}$ und $R^{21}$ | unabhängig voneinander Wasserstoff, $C_1$-$C_8$-Alkoxy, $C_1$-$C_8$-Alkyl, Phenyl oder Benzyl oder $R^{20}$ und $R^{21}$ zusammen mit dem sie tragenden Stickstoffatom einen 5- bis 6-gliedrigen gesättigten Stickstoffheterocyclus, der durch ein Sauerstoff-oder Schwefelatom unterbrochen sein kann, |
| $R^{22}$ | $C_1$-$C_4$-Alkyl, $C_3$-$C_4$-Alkenyl, $C_3$-$C_4$-Alkinyl oder $C_2$-$C_4$-Alkoxyalkyl, |
| $R^{23}$ | Wasserstoff oder Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein-oder mehrfach durch Amino, Halogen, Hydroxyl, Cyan, Nitro, Phenyl, $C_1$-$C_4$-Alkoxy, Polyethoxy mit 2 bis 6 Ethylenoxideinheiten, - $COOR^{27}$, $-COSR^{28}$, $-CONH_2$, $CON(C_1$-$C_4$-alkoxy)-$C_1$-$C_4$-alkyl, -CO-N-di-$C_1$-$C_4$-alkyl, CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, $-COOR^{29}$, $COSR^{30}$, - $CONH_2$, $-CON(C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl, |
| $R^{25}$ und $R^{26}$ | unabhängig voneinander $C_1$-$C_4$-Alkyl oder zusammen eine 3-bis 6-gliedrige Alkylenkette und |
| $R^{27}$, $R^{28}$, $R^{29}$ und $R^{30}$ | unabhängig voneinander Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkyl, $C_3$-$C_6$-Alkinyl oder $C_3$-$C_6$-Halogenalkinyl bedeuten. |

Die Substituenten $R^{15}$ und $R^{16}$ stehen bevorzugt für Halogen.

Bevorzugt sind herbizide Aryloxyphenoxypropionsäurederivate der Formel IV, worin Q für den Rest

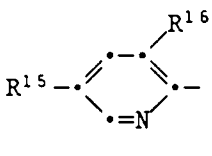 , 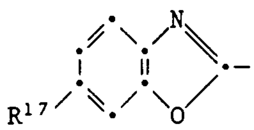 , 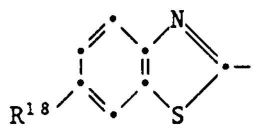 oder 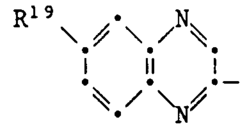

steht, wobei $R^{15}$ und $R^{16}$ Halogen bedeuten und $R^{17}$, $R^{18}$, $R^{19}$ und T die vorstehend für Formel IV angegebenen Bedeutungen haben.

Unter die Formel IV fallen insbesondere die folgenden herbiziden Aryloxyphenoxypropionsäurederivate:
2-[4-(3,5-Dichlorpyridin-2-yloxy)-phenoxy]-propionsäure-propargylester,
2-[4-(3,5-Dichlorpyridin-2-yloxy)-phenoxy]-thiopropionsäure-propargylester,
2-[4-(5-Chlor-3-fluorpyridin-2-yloxy)-phenoxy]-propionsäurepropargylester,
2-[4-(5-Chlor-3-fluorpyridin-2-yloxy)-phenoxy]-propionsäure-methylester,
2-[4-(5-Chlor-3-fluorpyridin-2-yloxy)-phenoxy]-propionsäure-butylester,
2-[4-(5-Trifluormethylpyridin-2-yloxy)-phenoxy]-propionsaure-butylester,
2-[4-(5-Trifluormethylpyridin-2-yloxy)-phenoxy]-propionsäure-methylester,
2-[4-(6-Chlorchinoxalin-2-yloxy)-phenoxy]-propionsäure-ethylester und
2-[4-(6-Chlorbenzoxazolin-2-yloxy)-phenoxy]-propionsäure-ethylester.

Die Aryloxyphenoxypropionsäurederivate der Formel IV können, bedingt durch die asymmetrische Substitution am $\alpha$-Kohlenstoffatom der Propionsäure in zwei unterschiedlichen stereoisomeren Formen vorliegen. Die Erfindung betrifft sowohl die Verwendung der Verbindungen der Formel I als Antidots für die Racemate der Verbindungen der Formel IV als auch für die optischen Isomeren der Verbindungen der Formel IV. Optische Isomere von Verbindungen der Formel IV sind unter anderem beschrieben in der US-Patentschrift US 4,505,743, welche D(+)-Isomere der Formel IV betrifft.
In besonders vorteilhafter Weise können die Verbindungen der Formel I auch bei den aus der EP-A-0 248 968 bekannt gewordenen R-Isomeren der Formel IV eingesetzt werden.
Herauszustellen ist hier der 2R-2-[4-(5-Chlor-3-fluor-pyrid-2-yloxy)-phenoxy]-propionsäurepropargylester.

Die N-Benzoyl-N-phenylalanine, deren kulturpflanzenschädigende Wirkung durch die Verbindungen der Formel I antagonisiert wird, entsprechen der Formel V

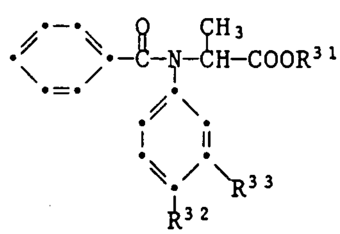 (V),

worin

| | |
|---|---|
| $R^{31}$ | Wasserstoff oder $C_1$-$C_4$-Alkyl und |
| $R^{32}$ und $R^{33}$ | unabhängig voneinander Fluor oder Chlor bedeutet. |

Als Einzelverbindungen der Formel V zu nennen sind:
N-Benzoyl-N-(3,4-dichlorphenyl)alanin-ethylester und
N-Benzoyl-N-(3-chlor-4-fluorphenyl)alanin-methylester.

Die Verbindungen der Formel V sind an sich bekannt (R. Wegler; Chemie der Pflanzenschutz- und Schädlingsbekämpfungsmittel Bd. 5, Springer Verlag, Berlin 1977, S. 194-195). Die Antidots der Formel I können mit Erfolg sowohl bei den racemischen Verbindungen der Formel V als auch bei deren Enantiomeren eingesetzt werden.

Die Antidots der Formel I eignen sich ganz besonders, um Kulturpflanzen vor den schädigenden Wirkungen der Herbizide der Formeln II, III, IV oder V zu schützen.

Die vorliegende Erfindung erstreckt sich auch auf ein Verfahren zum Schützen von Kulturpflanzen gegen die kulturpflanzenschädigende Wirkung von Herbiziden, welches darin besteht, dass man vor, während oder nach der Applikation des Herbizids die Kulturpflanze oder deren Lebensraum oder Samen

oder Stecklinge der Kulturpflanze mit einer antagonistisch wirksamen Menge einer Verbindung der Formel I behandelt.

Agrochemische Mittel, welche in einer gemeinsamen Formulierung neben dem Antidot der Formel I ein Herbizid, insbesondere ein Sulfonylharnstoffherbizid, ein N-Benzoyl-N-phenylalaninherbizid, ein Chloracetanilidherbizid oder ein Aryloxyphenoxypropionsäureherbizid enthalten, sind zum Einsatz als selektive Herbizide in Nutzpflanzenkulturen geeignet. Vorzugsweise enthalten die erfindungsgemässen herbiziden Mittel neben dem Antidot der Formel I als Herbizid einen Sulfonylharnstoff der Formel II, ein Chloracetanilid der Formel III, ein Aryloxyphenoxypropionsäurederivat der Formel IV oder ein N-Benzoyl-N-phenylalanin der Formel V. Bevorzugte Mischungspartner sind die vorstehend hervorgehobenen Antidots der Formel I und die für die Verwendung vorstehend hervorgehobenen Herbizide der Formeln II, III, IV und V.

Die angewendete Menge an Gegenmittel, sofern sie nicht auf die Samen gebeizt wird, schwankt zwischen etwa 0,01 und etwa 100 Gewichtsteilen pro Gewichtsteil Herbizid und liegt vorzugsweise im Bereich von 0,1 und etwa 10 Gewichtsteilen der Verbindung der Formel I je Gewichtsteil Herbizid. In der Praxis ermittelt man jeweils, von Fall zu Fall, das heisst je nach verwendetem Herbizid-Typ, welches Verhältnis in Bezug auf optimale Wirkung bei der speziellen Kulturpflanze das geeignetste ist.

Die Erfindung betrifft ferner Mittel, welche eine Verbindung der Formel I' mit ein oder mehreren Hilfsstoffen, Trägerstoffen oder Hilfs- und Trägerstoffen enthalten.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid und einer Verbindung der Formel 1 oder einem Mittel, welches diese Kombination enthält, behandelt werden. Die die Herbizid/Antidot-Kombination enthaltenden Mittel bilden ebenfalls einen Bestandteil der vorliegenden Erfindung.

Bei den zu bekämpfenden Unkräutern kann es sich sowohl um monokotyle wie um dikotyle Unkräuter handeln.

Als Unkräuter zu nennen sind insbesondere die Unkräuter der Gattungen Echinochloa, Rottboellia, Digitaria und Setaria.

Für die Verwendung der Verbindung der Formel I oder sie enthaltender Mittel zum Schützen von Kulturpflanzen gegen schädigende Wirkungen von Agrarchemikalien kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei etwa 0,1 bis 2,0 g Wirkstoff der Formel I (bei einer 25 %igen Formulierung: 0,4 g bis 8,0 g Spritzpulver) pro 1 kg Saatgut.

b) Beizung der Samen mit einem Emulsionskonzentrat des Wirkstoffs oder mit einer wässrigen Lösung des als Spritzpulver formulierten Wirkstoffs der Formel 1 nach Methode a) (Nassbeizung).

c) Beizung durch Tauchen des Saatguts in einer Brühe mit 10-1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 0,01 g bis 6,0 g, vorzugsweise 0,1 g bis 2,0 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Gegenmittel und Herbizid (gegenseitiges Mengenverhältnis zwischen 100:1 und 1:100, vorzugsweise 10:1 und 1:10) wird verwendet, wobei die Aufwandmengen an Herbizid und Safener jeweils 0,001 bis 10 kg pro Hektar betragen. Solche Tankmischung wird vorzugsweise vor oder nach der Aussaat appliziert und bei Anwendung vor der Aussaat 5 bis 10 cm tief in den noch nicht gesäten Boden eingearbeitet.

iii) Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten oder auch Verkapselungen verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die einen Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioktylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie zum Beispiel die Na- oder K-Salze der Oel- oder Stearinsäure, oder von natürlichen Fettsäuregemischen, die beispielsweise aus Kokosnuss- oder Tallöl gewonnen werden können, genannt. Ferner sind auch die Fettsäuremethyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, zum Beispiel das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 Kohlenstoffatomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensations-produktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenolpolyethoxyethanol, Polyethylenglykol und Oktylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 Kohlenstoffatomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, beispielsweise das Stearyltrimethylammoniumchlorid oder das Benzyldi(2-chlorethyl)ethyl-ammoniumbromid.

Die in der Formulierungstechnik gebräuchlichen Tenside sind u.a. in folgenden Publikationen beschrieben:

"1986 International Mc Cutcheon's Emulsifiers & Detergents", Glen Rock NJ USA, 1986",
H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die vorstehend offenbarten Formulierungsmöglichkeiten lassen sich nicht nur mit Antidots der Formel I anwenden, sondern im allgemeinen auch mit Wirkstoffgemischen aus Antidots der Formel I und Herbiziden, insbesondere Herbiziden der Formeln II, III, IV und V.

Die agrochemischen Zubereitungen enthalten in der Regel 0,1 bis 95 %, insbesondere 0,1 bis 80 % Wirkstoff der Formel I oder eines Gemisches von Antidot und Herbizid, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

| Emulgierbare Konzentrate | |
|---|---|
| Aktives Wirkstoffgemisch | 1 bis 20 %, bevorzugt 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 % |

| Stäube: | |
|---|---|
| Aktives Wirkstoffgemisch: | 0,1 bis 10 %, vorzugsweise 0,1 bis 1 % |
| festes Trägermittel: | 99,9 bis 90 %, vorzugsweise 99,9 bis 99 %. |

| Suspension-Konzentrate | |
|---|---|
| Aktives Wirkstoffgemisch: | 5 bis 75 %, vorzugsweise 10 bis 50 % |
| Wasser: | 94 bis 25 %, vorzugsweise 88 bis 30 % |
| oberflächenaktives Mittel: | 1 bis 40 %, vorzugsweise 2 bis 30 % |

| Benetzbare Pulver | |
|---|---|
| Aktives Wirkstoffgemisch: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

| Granulate | |
|---|---|
| aktives Wirkstoffgemisch: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,25 bis 5 kg AS/ha.

Die Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die erfindungsgemäss verwendbaren neuen Verbindungen der Formel I'

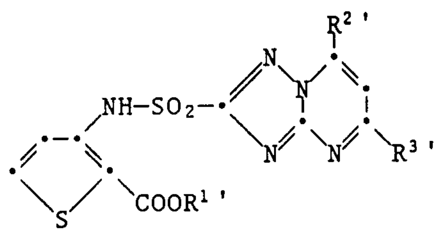

(I'),

worin
$R^{1'}$ $C_1$-$C_6$-Alkyl oder $C_3$-$C_6$-Cycloalkyl und
$R^{2'}$ und $R^{3'}$ unabhängig voneinander Wasserstoff, $C_1$-$C_3$-Alkyl, Trifluormethyl oder Cyclopropyl bedeuten, mit der Massgabe, dass mindestens eines der Symbole $R^{2'}$ und $R^{3'}$ für Cyclopropyl steht, lassen sich herstellen, indem man eine Verbindung der Formel VI

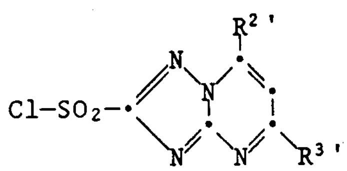

(VI),

worin $R^{2'}$ und $R^{3'}$ unabhängig voneinander Wasserstoff, $C_1$-$C_3$-Alkyl, Trifluormethyl oder Cyclopropyl bedeuten, mit der Massgabe, dass mindestens eines der Symbole $R^{2'}$ und $R^{3'}$ für Cyclopropyl steht, mit einer Verbindung der Formel VII

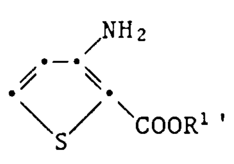

(VII),

worin $R^{1'}$ $C_1$-$C_6$-Alkyl oder $C_3$-$C_6$-Cycloalkyl bedeutet, umsetzt.

Die Umsetzung erfolgt zweckmässigerweise in einem Temperaturbereich von 5°C bis 25°C und in Gegenwart einer Base, wie beispielsweise Pyridin oder einem tertiären Amin wie 4-Dimethylaminopyridin, N-Methylmorpholin oder Triäthylamin. Die Umsetzung wird bevorzugt in einem reaktionsinerten Lösungsmittel durchgeführt, wie beispielsweise Pyridin, Dichlormethan oder Acetonitril. Zweckmässigerweise setzt man die Reaktanden der Formeln VI und VII in äquimolarem Verhältnis zueinander ein oder verwendet einen geringen Ueberschuss der Verbindung der Formel VI.

Die Zwischenprodukte der Formel VII sind bekannt oder lassen sich analog bekannten Verbindungen herstellen.

Die Zwischenprodukte der Formel VI sind erhältlich durch Behandeln einer Verbindung der Formel VIII

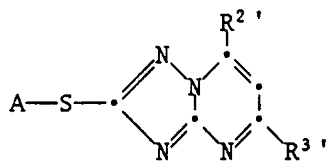

(VIII),

worin $R^{2'}$ und $R^{3'}$ unabhängig voneinander Wasserstoff, $C_1$-$C_3$-Alkyl, Trifluormethyl oder Cyclopropyl bedeuten, mit der Massgabe, dass mindestens eines der Symbole $R^{2'}$ und $R^{3'}$ für Cyclopropyl steht, und A Wasserstoff oder Benzyl bedeutet, in wässrigem, saurem Medium mit Chlor. Als Säure verwendet man organische Säure, insbesondere Essigsäure, oder vorzugsweise anorganische Säure, vor allem Salzsäure. Die Behandlung mit Chlor erfolgt zweckmässigerweise bei Temperaturen im Bereich von -25°C bis 20°G, bevorzugt bei -15°C bis 0°C. Vorzugsweise erfolgt die Behandlung mit Chlor unter Zusatz von Dichlormethan zum Medium.

Die Zwischenprodukte der Formel VIII sind herstellbar durch Umsetzung einer Verbindung der Formel IX

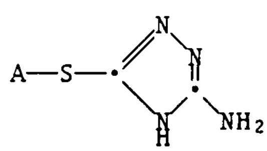

(IX),

worin A Wasserstoff oder Benzyl bedeutet, mit einer Verbindung der Formel X

$R_b—CO—CH_2—R_a$ (X),

worin i) $R_a$ für die Gruppe -CO-$R_c$ steht und eines der Symbole $R_b$ und $R_c$ für $R^{2'}$ und das andere für $R^{3'}$ steht oder ii) $R_a$ für die Gruppe -CH($OR_d$)$_2$ steht und $R_b$ für $R^{2'}$ oder $R^{3'}$ und $R_d$ für Methyl oder Aethyl steht.

Die Zwischenprodukte der Formeln IX und X sind bekannt oder lassen sich analog bekannten Methoden herstellen. Die Verbindung der Formel IX, worin A für Benzyl steht, lässt sich beispielsweise auf an sich bekannte Weise durch Umsetzung der Verbindung der Formel IX, in welcher A für Wasserstoff steht, mit Benzylchlorid herstellen.

Die Umsetzung einer Verbindung der Formel IX mit einer Verbindung der Formel X erfolgt vorteilhafterweise, indem man zunächst eine Verbindung der Formel IX unter Erhitzen in wenig Eisessig löst und nach Zugabe der Verbindung der Formel X das Reaktionsgemisch auf Rückflusstemperatur erhitzt.

Die für die Herstellung von Verbindungen der Formel I' entwickelten Zwischenprodukte der Formeln VI und VIII sind neu und bilden jeweils ebenfalls einen Gegenstand der vorliegenden Erfindung.

Die Verbindungen der Formel I, welche nicht unter Formel I' fallen, lassen sich analog den für Verbindungen der Formel I' angegebenen Methoden herstellen.

Die folgenden Beispiele dienen der Erläuterung der Erfindung. Sie schränken die Erfindung nicht ein.

Beispiel H1: Herstellung von N-(5-Methyl-7-cyclopropyl-1,2,4-triazolo[1,5-a]pyrimidin-2-ylsulfonyl)-3-aminothiophen-2-carbonsäuremethylester

a) 5-Methyl-7-cyclopropyl-1,2,4-triazolo[1,5-a]pyrimidin-2-yl-sulfochlorid

35 g 2-Benzylthio-5-methyl-7-cyclopropyl-1,2,4-triazolo[1,5-a]pyrimidin in 200 ml Dichlormethan werden mit 300 ml Wasser und 20 ml konzentrierter Salzsäure verrührt. In das Gemisch werden bei 0 °C bis -3 °C 33,5 g Chlorgas eingeleitet. Nach ca. 30 Minuten ist die Einleitung beendet und das Reaktionsgemisch wird noch ca. 20 Minuten ohne Kühlung nachgerührt. Nach Verdünnen mit Wasser wird die organische Phase abgetrennt, über Natriumsulfat getrocknet und am Vakuum eingedampft. Das erhaltene Oel wird mit mehreren Portionen Petroläther gewaschen. Nach dem Trocknen liegt 5-Methyl-7-cyclopropyl-1,2,4-triazolo-[1,5-a]pyrimidin-2-yl-sulfochlorid als Rohprodukt vor, welches für die weiteren Umsetzungen geeignet ist. Ausbeute: 28 g dunkles Oel.

b) N-(5-Methyl-7-cyclopropyl-1,2,4-triazolo[1,5-a]pyrimidin-2-ylsulfonyl)-3-aminothiophen-2-carbonsäuremethylester

14 g N-(5-Methyl-7-cyclopropyl-1,2,4-triazolo[1,5-a]pyrimidin-2-yl-sulfochlorid (Rohprodukt gemäss Teil a) in 15 ml Pyridin werden mit 7,9 g 3-Aminothiophen-2-carbonsäuremethylester verrührt. Nach Lösung unter leicht exothermer Reaktion färbt sich das Reaktionsgemisch dunkel. Nach längerem Stehenlassen bildet sich ein leichter Niederschlag. Das Reaktionsgemisch wird nun mit ca. 100 ml Essigester/Hexan 1:1 und 300 ml Wasser verdünnt und mit Soda stark alkalisch gemacht. Die wässrige Phase wird abgetrennt und mit Salzsäure angesäuert. Der ausfallende Niederschlag wird gesammelt und getrocknet (19 g). Das Produkt wird aus Aceton/Hexan umkristallisiert. Man erhält 14,8 g N-(5-Methyl-7-cyclopropyl-1,2,4-triazolo-[1,5-a]pyrimidin-2-ylsulfonyl)-3-aminothiophen-2-carbonsäuremethylester. Smp. 185-187 °C (Zers.).

Beispiel H2: Herstellung von N-(5-Cyclopropyl-7-methyl-1,2,4-triazolo[1,5-a]pyrimidin-2-ylsulfonyl)-3-aminothiophen-2-carbonsäuremethylester

a) 5-Cyclopropyl-7-methyl-1,2,4-triazolo[1,5-a]pyrimidin-2-yl-sulfochlorid

14 g 2-Benzylthio-5-cyclopropyl-7-methyl-1,2,4-triazolo[1,5-a]pyrimidin in 200 ml Dichlormethan und 200 ml Wasser werden mit 10 ml konzentrierter Salzsäure vermischt und kräftig gerührt. Bei 0 °C bis -3 °C werden im Zeitraum von ca. 20 Minuten 13,5 g Chlorgas eingeleitet. Nach etwa halbstündigem Nachrühren ohne Kühlung wird die organische Phase abgetrennt, mit Wasser nachgewaschen und über Natriumsulfat getrocknet. Nach dem Eindampfen am Vakuum verbleibt ein dunkles Oel, welches mehrmals mit Petroläther durchgerührt wird. Der Petroläther wird abdekantiert und der erhaltene zähe Rückstand getrocknet. Man erhält 11,5 g 5-Cyclopropyl-7-methyl-1,2,4-triazolo[1,5-a]pyrimidin-2-yl-sulfochlorid als dunkles Oel (Rohprodukt), welches für die weiteren Umsetzungen geeignet ist. Das Protonenresonanzspektrum bestätigt die Konstitution der erhaltenen Verbindung.

b) N-(5-Cyclopropyl-7-methyl-1,2,4-triazolo[1,5-a]pyrimidin-2-ylsulfonyl)-3-aminothiophen-2-carbonsäuremethylester

5,7 g 5-Cyclopropyl-7-methyl-1,2,4-triazolo[1,5-a]pyrimidin-2-yl-sulfochlorid (Rohprodukt gemäss Teil a) werden in 15 ml Pyridin mit 3,5 g 3-Aminothiophen-2-carbonsäuremethylester verrührt. Die weitere Behandlung des Reaktionsgemisches erfolgt wie im Beispiel 1 (Teil b) beschrieben. Man erhält 4,8 g N-(5-Cyclopropyl-7-methyl-1,2,4-triazolo[1,5-a]pyrimidin-2-ylsulfonyl)-3-aminothiophen-2-carbonsäuremethylester. Smp. 133-134,5 °C.

Beispiel H3: Herstellung von 2-Benzylthio-5-methyl-7-cyclopropyl-1,2,4-triazolo[1,5-a]pyrimidin (Verbindung A) und 2-Benzylthio-5-cyclopropyl-7-methyl-1,2,4-triazolo[1,5-a]pyrimidin (Verbindung B)

41,5 g 3-Amino-5-benzylthio-1,2,4-triazol werden in wenig heissem Eisessig gelöst und zusammen mit 30 g 1-Cyclopropyl-1,3-butandion am Rückfluss erhitzt. Nach ca. 1 Stunde wird die Reaktionslösung am Vakuum eingeengt und der Rückstand wird mit Wasser versetzt. Das ausgefallene dunkle Harz wird mit Essigester extrahiert und die Essigesterphase wird über Natriumsulfat getrocknet und eingedampft. Der Rückstand wird an einer Kieselgelsäule (Laufmittel: Essigester/Hexan 1:1) gereinigt. Man erhält die Titelverbindungen A und B:

1. Fraktion 20 g Smp. 101-103 °C (Verbindung B)
2. Fraktion 39 g Smp. 84-86 °C (Verbindung A)

Analog zu den beschriebenen Arbeitsweisen werden auch die nachfolgend zusammen mit Verbindungen der vorgängigen Beispiele genannten Verbindungen der Formel I (Endprodukte, Tabelle 1) sowie der Formeln VI (Zwischenprodukte, Tabelle 2) und VIII (Zwischenprodukte, Tabelle 3) hergestellt:

Tabelle 1

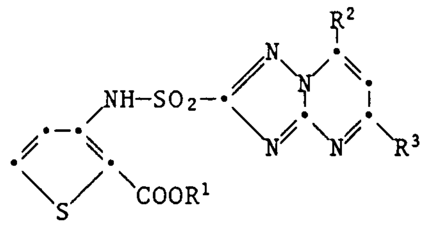

| Verb. Nr. | $R^1$ | $R^2$ | $R^3$ | Phys. Daten/Smp. |
|---|---|---|---|---|
| 1.01 | $CH_3$ | Cyclopropyl | $CH_3$ | 185-187°C |
| 1.02 | $CH_3$ | $CH_3$ | Cyclopropyl | 133-134°C |
| 1.03 | $CH_3$ | H | $CH_3$ | 213-214°C |
| 1.04 | $CH_3$ | $CH_3$ | $CH_3$ | 164-165°C |
| 1.05 | $C_2H_5$ | $CH_3$ | $CH_3$ | 181-183°C |
| 1.06 | $C_6H_{13}$ | $CH_3$ | $CH_3$ | |
| 1.07 | $C_4H_9$ | $CH_3$ | $CH_3$ | |
| 1.08 | $C_2H_5$ | Cyclopropyl | $CH_3$ | |
| 1.09 | $C_2H_5$ | $CH_3$ | Cyclopropyl | |
| 1.10 | $C_2H_5$ | H | Cyclopropyl | |
| 1.11 | $C_2H_5$ | Cyclopropyl | H | |
| 1.12 | $CH_3$ | H | Cyclopropyl | |
| 1.13 | $CH_3$ | Cyclopropyl | H | |
| 1.14 | $CH_3$ | $CH_3$ | H | |
| 1.15 | $C_3H_7(i)$ | $CH_3$ | $CH_3$ | 164-167°C |
| 1.16 | $C_2H_5$ | H | $CH_3$ | |
| 1.17 | $CH_3$ | $CH_3$ | H | 158-160°C |
| 1.18 | $C_2H_5$ | H | $CH_3$ | 137-139,5°C |
| 1.19 | $CH_3$ | Cyclopropyl | Cyclopropyl | 184-186°C |
| 1.20 | $CH_3$ | $CF_3$ | Cyclopropyl | |

Tabelle 2

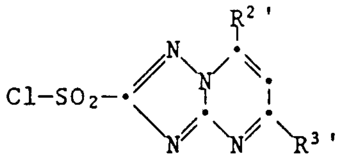

| Verb. Nr. | $R^{2'}$ | $R^{3'}$ | Physikal. Daten |
|---|---|---|---|
| 2.01 | H | Cyclopropyl | |
| 2.02 | Cyclopropyl | H | |
| 2.03 | $CH_3$ | Cyclopropyl | Oel |
| 2.04 | Cyclopropyl | $CH_3$ | Oel |
| 2.05 | Cyclopropyl | Cyclopropyl | 127,5-130°C |
| 2.06 | $CF_3$ | Cyclopropyl | 78-81°C |

Tabelle 3

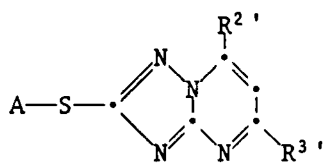

| Verb. Nr. | A | $R^{2'}$ | $R^{3'}$ | Phys. Daten/Smp. |
|---|---|---|---|---|
| 3.01 | H | H | Cyclopropyl | |
| 3.02 | H | Cyclopropyl | H | |
| 3.03 | Benzyl | Cyclopropyl | H | 82-86°C |
| 3.04 | Benzyl | Cyclopropyl | $CH_3$ | 84-86°C |
| 3.05 | Benzyl | $CH_3$ | Cyclopropyl | 101-103°C |
| 3.06 | Benzyl | H | Cyclopropyl | 112-114°C |
| 3.07 | Benzyl | $CF_3$ | Cyclopropyl | 123-124°C |
| 3.08 | Benzyl | Cyclopropyl | Cyclopropyl | 96-98°C |

## Biologische Beispiele

### B1. Safeningwirkung in Wasserreis

Zur Ueberprüfung der Safeningwirkung werden Samen von Reis (Sorte YAMABIKO, NIHONBARE) in Saatschalen, welche mit normal bewässerter Gartenerde beschickt sind, gesät. Im 2-Blattstadium werden die Pflanzen in Behälter mit stark sumpfiger Erde transplantiert. Der Wasserstand wird mit zunehmendem Wachstum angehoben.

Die Kultur der Pflanzen erfolgt im Gewächshaus unter angepassten Temperatur- und Lichtverhältnissen. Das Giessen resp. Düngen der Pflanzen erfolgt nach Bedarf.

Der Safener wird der sumpfigen Erde 7 Tage vor der Verpflanzung zugegeben und das Herbizid wird 3 Tage nach der Verpflanzung als Einlauf ins Wasser appliziert.

Zur Erfassung der Safeningwirkung (Schutzwirkung) wird 10 Tage nach der Applikation des Herbizids die an den Pflanzen auftretende allgemeine Schädigung erfasst (Phytotoxizität 0 %: keine Schädigung, Zustand wie unbehandelte Kontrolle; Phytotoxizität 100 %: maximale Schädigung). Aus der Differenz der durch ausschliessliche Applikation des Herbizids verursachten Phytotoxizität und der bei Applikation von Herbizid und Safener auftretenden Phytotoxizität ergibt sich die in % ausgedrückte Schutzwirkung.

Die Ergebnisse für das Herbizid der Formel II

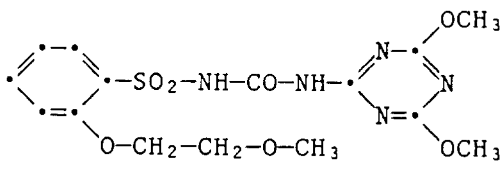

(HE-1)

N-[2-(2-Methoxyethoxy)-phenylsulfonyl]-N'-4,6-dimethoxy-1,3,5-triazin-2-yl-harnstoff (bekannt aus EP-A-0 044 807 und EP-A-0 224 441) (Herbizid HE-1) und den Safener der Formel I N-(5,7-Dimethyl-1,2,4-triazolo-[1,5-a]pyrimidin-2-yl-sulfonyl)-3-aminothiophen-2-carbonsäuremethylester (Safener S-1) sind in Tabelle 4 dargestellt:

Tabelle 4

| Aufwandmengen | Schutzwirkung in % | |
|---|---|---|
| 100 ppm S-1; 260 g/ha HE-1 | Reis der Sorte | |
| | "YAMABIKO" 20 | "NIHONBARE" 30 |

B2. Safeningwirkung in Wasserreis (Tankmix)

Zur Ueberprüfung der Safeningwirkung werden Samen von Reis (Sorte S-201) in Töpfen von 9 x 9 cm (Durchmesser x Höhe) in sumpfiger Erde angezogen. Die Kultur der Pflanzen erfolgt im Gewächshaus unter angepassten Temperatur-und Lichtverhältnissen. Das Giessen resp. Düngen der Pflanzen erfolgt nach Bedarf.

Safener und Herbizid werden gemeinsam als Tankmix mit einer Wasseraufwandmenge von 550 l/ha direkt nach der Saat (Pre) oder 3 Tage nach der Saat (3 DAS) appliziert.

Zur Erfassung der Safeningwirkung (Schutzwirkung) wird 24 Tage nach Applikation der Wirkstoffmischung die an den Pflanzen auftretende allgemeine Schädigung erfasst (Phytotoxizität 0 %: keine Schädigung, Zustand wie unbehandelte Kontrolle; Phytotoxizität 100 %: maximale Schädigung). Aus der Differenz der durch ausschliessliche Applikation des Herbizids verursachten Phytotoxizität und der bei Applikation von Herbizid und Safener auftretenden Phytotoxizität ergibt sich die in % ausgedrückte Schutzwirkung.

Als Wirkstoffe werden die im Beispiel BI eingesetzten Substanzen (Herbizid HE-1 und Safener S-1) verwendet. Die Ergebnisse zeigt Tabelle 5.

Tabelle 5

| Applikationszeitpunkt | PRE | | 3 DAS | |
|---|---|---|---|---|
| HE-1 Aufwandmenge [g/ha] | 30 | | 30 | |
| S-1 Aufwandmenge [g/ha] | 120 | 60 | 120 | 60 |
| Schutzwirkung in % bei Reis der Sorte "S-201" | 40 | 45 | 60 | 60 |

B3. Safeningwirkung in Getreide (Tankmix)

Zur Ueberprüfung der Safeningwirkung werden Samen von Weizen (Sorte BESSO) und Gerste (Sorte CORNEL) in Töpfen von 11 cm Durchmesser in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf.

Safener und Herbizid werden gemeinsam als Tankmix mit einer Wasseraufwandmenge von 550 l/ha im Nachauflauf (post) appliziert.

Zur Erfassung der Safeningwirkung (Schutzwirkung) wird 18 Tage nach der Applikation der Wirkstoffmischung die an den Pflanzen auftretende allgemeine Schädigung erfasst (Phytotoxizität 0 %: keine Schädigung, Zustand wie unbehandelte Kontrolle; Phytotoxizität 100 %: maximale Schädigung). Aus der Differenz der durch ausschliessliche Applikation des Herbizids verursachten Phytotoxizität und der bei Applikation von Herbizid und Safener auftretenden Phytotoxizität ergibt sich die in % ausgedrückte Schutzwirkung.

Die Ergebnisse für die folgenden Herbizide der Formel IV

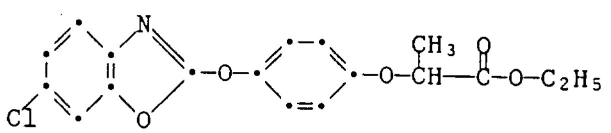

(HE-2)

2-[4-(6-Chlorbenzoxazol-2-yloxy)phenoxy]propionsäureethylester (The Pesticide Manual; $8^{th}$ Ed. British Crop Protection Council, Thornton Heath, 1987, Verb. No. 6160).

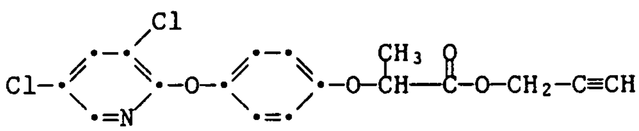

(HE-3)

2-[4-(3,5-Dichlorpyridin-2-yloxy)phenoxy]-propionsäurepropargylester (bekannt aus der EP-A-0 003 114) und

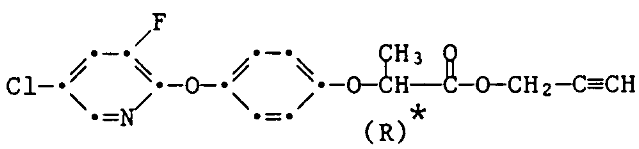

(HE-4)

2R-2-[4-(5-Chlor-3-fluorpyridin-2-yloxy)phenoxy]-propionsäurepropargylester (bekannt aus EP-A-0 248 968) und den Safener S-1 aus Beispiel 1 sind in Tabelle 6 zusammengestellt:

Tabelle 6

| Herbizid | HE-2 | | HE-3 | | HE-4 | |
|---|---|---|---|---|---|---|
| Aufwandmenge [g/ha] | 1000 | | 1000 | | 120 | |
| Safener S-1 Aufwandmenge [g/ha] | 2000 | 500 | 2000 | 500 | 2000 | 500 |
| Schutzwirkung in % bei | | | | | | |
| Weizen der Sorte "BESSO" | 50 | 55 | 73 | 48 | 60 | 35 |
| Gerste der Sorte "CORNEL" | 60 | 70 | 33 | 28 | - | - |

B4. Safeningwirkung in Reis (Tankmix)

Zur Ueberprüfung der Safeningwirkung werden Samen von Reis (Sorte NEWBONNET) in Töpfen von 11 cm Durchmesser in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf. Das Ungras Echinochloa wird nach dem gleichen Verfahren angezogen.

Safener und Herbizid werden gemeinsam als Tankmix mit einer Wasseraufwandmenge von 550 l/ha im Nachauflauf (post) appliziert, wobei verschiedene Wachstumsstadien der Testpflanzen verwendet werden.

Zur Erfassung der Safeningwirkung (Schutzwirkung) wird 23 Tage nach der Applikation der Wirkstoffmischung die an den Pflanzen auftretende allgemeine Schädigung erfasst (Phytotoxizität 0 %: keine Schädigung, Zustand wie unbehandelte Kontrolle; Phytotoxizität 100 %: maximale Schädigung). Aus der Differenz der durch ausschliessliche Applikation des Herbizids verursachten Phytotoxizität und der bei Applikation von Herbizid und Safener auftretenden Phytotoxizität ergibt sich die in % ausgedrückte Schutzwirkung.

Die Ergebnisse für das Herbizid HE-4 (gemäss Beispiel B3) und den Safener S-1 (gemäss Beispiel B1) sind in Tabelle 7 zusammengestellt:

Tabelle 7

| Applikationszeitpunkt | 1-2-Blattstadium | | | 2-3-Blattstadium | | | 4-Blattstadium | | |
|---|---|---|---|---|---|---|---|---|---|
| Herbizid HE-4 Aufwandmenge [g/ha] | 60 | | | 60 | | | 60 | | |
| Safener S-1 Aufwandmenge [g/ha] | 480 | 120 | 30 | 480 | 120 | 30 | 480 | 120 | 30 |
| Schutzwirkung in % bei | | | | | | | | | |
| Reis der Sorte "NEWBONNET" | 60 | 35 | 40 | 60 | 60 | 50 | 80 | 45 | 40 |
| Echinochloa crus galli | 2 | 0 | 0 | 2 | 0 | 0 | 5 | 1 | 0 |

B5. Safeningwirkung in Reis (Tankmix)

Zur Ueberprüfung der Safeningwirkung werden Samen von Reis (Sorten NEWBONNET, BALILLA, JUNG-WEON, PB 56, IR54, YAMABIKO, STARBONNET) in Töpfen von 11 cm Durchmesser in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf. Die Unkräuter Echinochloa, Rottboellia und Digitaria werden nach dem gleichen Verfahren angezogen.

Safener und Herbizid werden gemeinsam als Tankmix mit einer Wasseraufwandmenge von 550 l/ha im Nachauflauf (post) appliziert.

Zur Erfassung der Safeningwirkung (Schutzwirkung) wird 23 Tage nach der Applikation der Wirkstoffmischung die an den Pflanzen auftretende allgemeine Schädigung erfasst (Phytotoxizität 0 %: keine Schädigung, Zustand wie unbehandelte Kontrolle; Phytotoxizität 100 %: maximale Schädigung). Aus der Differenz der durch ausschliessliche Applikation des Herbizids verursachten Phytotoxizität und der bei Applikation von Herbizid und Safener auftretenden Phytotoxizität ergibt sich die in % ausgedrückte Schutzwirkung.

Für die Herbizid/Safenerkombination (gemäss Beispiel B4) erhält man die in Tabelle 8 angegebenen Ergebnisse:

Tabelle 8

| Herbizid HE-4 Aufwandmenge [g/ha] | 60 | | |
|---|---|---|---|
| Safener S-1 Aufwandmenge [g/ha] | 480 | 120 | 30 |
| Schutzwirkung in % | | | |
| Reis der Sorten | | | |
| "NEWBONNET" | 50 | 40 | 35 |
| "BALILLA" | 90 | 65 | 50 |
| "JUNG-WEON" | 55 | 60 | 60 |
| "PB 56" | 60 | 50 | 45 |
| "IR 54" | 45 | 40 | 35 |
| "YAMABIKO" | 60 | 20 | 25 |
| "STARBONNET" | 60 | 45 | 35 |
| Echinochloa c.g. | 0 | 0 | 0 |
| Rottboellia exaltata | 0 | 0 | 0 |
| Digitaria sang. | 8 | 8 | 3 |

B6. Safeningwirkung in Reis (Tankmix)

Zur Ueberprüfung der Safeningwirkung werden Samen von Reis (Sorte S-201) in Töpfen von 12 cm Durchmesser in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf. Das Ungras Setaria italica wird nach dem gleichen Verfahren angezogen.

Safener und Herbizid werden gemeinsam als Tankmix mit einer Wasseraufwandmenge von 550 l/ha im Nachauflauf (post) appliziert, wobei Reis im 1-2-Blattstadium und Setaria im 3-Blattstadium mit dem Tankmix behandelt werden.

Zur Erfassung der Safeningwirkung (Schutzwirkung) wird 10 Tage nach der Applikation der Wirkstoffmischung die an den Pflanzen auftretende allgemeine Schädigung erfasst (Phytotoxizität 0 %: keine Schädigung, Zustand wie unbehandelte Kontrolle; Phytotoxizität 100 %: maximale Schädigung). Aus der Differenz der durch ausschliessliche Applikation des Herbizids verursachten Phytotoxizität und der bei Applikation von Herbizid und Safener auftretenden Phytotoxizität ergibt sich die in % ausgedrückte Schutzwirkung.

Als Herbizide der Formel IV werden neben den Herbiziden HE-2 und HE-4 (gemäss Beispiel B3) die Herbizide

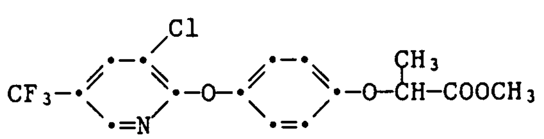 (HE-5)

2-[4-(3-Chlor-5-trifluormethyl-pyridin-2-yl-oxy)phenoxy]-propionsäuremethylester (The Pesticide Manual, 8th Ed.; British Crop Protection Council, Thornton Heath, 1987, Verb. No. 7010)
und

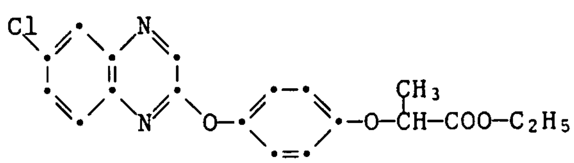 (HE-6)

2-[4-(6-Chlorchinoxalin-2-yloxy)phenoxy]-propionsäureethylester (The Pesticide Manual; 8th Ed., The British Crop Protection Council, Thornton Heath, 1987, Verb. No. 10 565)
und als Herbizid der Formel III

das Herbizid

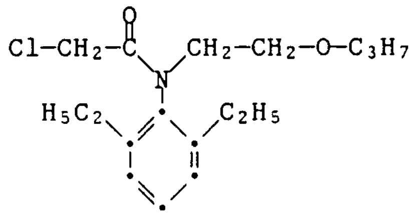

(HE-7)

2-Chlor-2',6'-diethyl-N-(2-propoxyethyl)-acetanilid (The Pesticide Manual; 8th Ed., The British Crop Protection Council, Thornton Heath, 1987, Verb. No. 9970)
in Kombination mit dem aus Beispiel 1 bekannten Safener S-1 verwendet.

Die Ergebnisse sind in Tabelle 9 zusammengefasst.

Tabelle 9

| Herbizid | HE-2 | HE-4 | HE-5 | HE-6 | HE-7 |
|---|---|---|---|---|---|
| Aufwandmenge [g/ha] | 128 | 64 | 128 | 64 | 512 |
| Safener S-1 Aufwandmenge [g/ha] | 100 | 100 | 100 | 100 | 100 |
| Schutzwirkung in % bei | | | | | |
| Reis der Sorte "S-201" | 35 | 20 | 25 | 90 | 35 |
| Setaria italica | 0 | 7 | 1 | 2 | 0 |

B7. Safeningwirkung in Wasserreis (Tankmix)

Zur Ueberprüfung der Safeningwirkung werden Samen von Reis (Sorte NEWBONNET) in Töpfen von 9 x 9 cm (Durchmesser x Höhe) in sumpfiger Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf.

Safener und Herbizid werden gemeinsam als Tankmix mit einer Wasseraufwandmenge von 550 l/ha im Nachauflauf (post) appliziert, wobei die Reispflanzen im 1-2-Blattstadium behandelt werden.

Zur Erfassung der Safeningwirkung (Schutzwirkung) wird 21 Tage nach Applikation der Wirkstoffmischung die an den Pflanzen auftretende allgemeine Schädigung erfasst (Phytotoxizität 0 %: keine Schädigung, Zustand wie unbehandelte Kontrolle; Phytotoxizität 100 %: maximale Schädigung). Aus der Differenz der durch ausschliessliche Applikation des Herbizids verursachten Phytotoxizität und der bei Applikation von Herbizid und Safener auftretenden Phytotoxizität ergibt sich die in % ausgedrückte Schutzwirkung.

Als Wirkstoffe werden das im Beispiel B3 eingesetzte Herbizid HE-4 und die folgenden Safener der Formel I verwendet:
N-(5-Methyl-1,2,4-triazolo[1,5-a]pyrimidin-2-ylsulfonyl)-3-aminothiophen-2-carbonsäuremethylester (S-2),
N-(5-Methyl-7-cyclopropyl-1,2,4-triazolo[1,5-a]pyrimidin-2-ylsulfonyl)-3-aminothiophen-2-carbonsäuremethylester (S-3),
N-(5-Cyclopropyl-7-methyl-1,2,4-triazolo[1,5-a]pyrimidin-2-ylsulfonyl)-3 aminothiophen-2-carbonsäuremethylester (S-4) und der im Beispiel B1 eingesetzte Safener S-1. Die Ergebnisse zeigt Tabelle 10.

Tabelle 10

| HE-4 Aufwandmenge [g/ha] | 120 | | | | 60 | | | |
|---|---|---|---|---|---|---|---|---|
| S-2 Aufwandmenge [g/ha] | 480 | 240 | 120 | | 480 | 240 | 120 | |
| Schutzwirkung in %: | 37 | 25 | 25 | | 25 | 25 | 37 | |
| S-3 oder S-4 oder S-1 Aufwandmenge [g/ha] | 400 | 100 | 25 | 6 | 400 | 100 | 25 | 6 |
| S-3, Schutzwirkung in %: | 50 | 37 | 12 | 12 | 50 | 50 | 25 | 12 |
| S-4, Schutzwirkung in %: | 33 | 12 | 12 | 0 | 60 | 55 | 30 | 25 |
| S-1, Schutzwirkung in %: | 60 | 40 | 40 | 20 | 60 | 60 | 45 | 30 |

B8. Safeningwirkung in Wasserreis (Tankmix)

Zur Ueberprüfung der Safeningwirkung werden Samen von Reis (Sorte S-201) in Töpfen von 9 x 9 cm (Durchmesser x Höhe) in sumpfiger Erde angezogen. Die Kultur der Pflanzen erfolgt im Gewächshaus unter angepassten Temperatur-und Lichtverhältnissen. Das Giessen resp. Düngen der Pflanzen erfolgt nach Bedarf.

Safener und Herbizid werden gemeinsam als Tankmix mit einer Wasseraufwandmenge von 550 l/ha vor dem Auflaufen der Pflanzen (Pre) appliziert.

Zur Erfassung der Safeningwirkung (Schutzwirkung) wird 21 Tage nach Applikation der Wirkstoffmischung die an den Pflanzen auftretende allgemeine Schädigung erfasst (Phytotoxizität 0 %: keine Schädigung, Zustand wie unbehandelte Kontrolle; Phytotoxizität 100 %: maximale Schädigung). Aus der Differenz der durch ausschliessliche Applikation des Herbizids verursachten Phytotoxizität und der bei Applikation von Herbizid und Safener auftretenden Phytotoxizität ergibt sich die in % ausgedrückte Schutzwirkung.

Als Wirkstoffe werden das im Beispiel B6 eingesetzte Herbizid HE-7 und der Safener der Formel I N-(5,7-Dimethyl-1,2,4-triazolo[1,5-a]pyrimidin-2-yl-sulfonyl)-3-aminothiophen-2-carbonsäureethylester (S-5) verwendet. Die Ergebnisse zeigt Tabelle 11.

Tabelle 11

| HE-7 Aufwandmenge [kg/ha] | 0,5 | | | 0,25 | | |
|---|---|---|---|---|---|---|
| S-5 oder S-2 Aufwandmenge [kg/ha] | 0,5 | 0,25 | 0,125 | 0,5 | 0,25 | 0,125 |
| S-5, Schutzwirkung in %: | 37 | 25 | 25 | 25 | 25 | 25 |
| S-2, Schutzwirkung in %: | 25 | 25 | 25 | 37 | 25 | 50 |

Formulierungsbeispiele

Die in den Formulierungsbeispielen verwendeten Bezeichnungen für Herbizide und Safener entsprechen denen der vorstehenden biologischen Beispiele.

Formulierungsbeispiele für Herbizid-Safener-Gemische

| F1 Spritzpulver | a) | b) | c) | d) |
|---|---|---|---|---|
| Herbizid HE-1 | 10 % | 20 % | 5 % | 30 % |
| Safener S-1 | 10 % | 40 % | 15 % | 30 % |
| Na-Ligninsulfonat | 5 % | 5 % | 5 % | 5 % |
| Na-Laurylsulfat | 3 % | - | 3 % | - |
| Na-Diisobutylnaphthalinsulfonat | - | 6 % | - | 6 % |
| Octylphenolpolyäthylenglykoläther 7-8 Mol AeO) | - | 2 % | - | 2 % |
| Hochdisperse Kieselsäure | 5 % | 27 % | 5 % | 27 % |
| Kaolin | 67 % | - | 67 % | - |

Das Wirkstoffgemisch wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F2 Emulsions-Konzentrat | a) | b) | c) |
|---|---|---|---|
| Herbizid HE-7 | 5 % | 5 % | 12 % |
| Safener S-1 | 5 % | 20 % | 13 % |
| Octylphenolpolyäthylenglykoläther (4-5 Mol AeO) | 3 % | 3 % | 3 % |
| Ca-Dodecylbenzolsulfonat | 3 % | 3 % | 2 % |
| Ricinusölpolyglykoläther (36 Mol AeO) | 4 % | 4 % | 4 % |
| Cyclohexanon | 30 % | 30 % | 31 % |
| Xylolgemisch | 50 % | 35 % | 35 % |

Aus diesen Konzentraten können durch Verdünnen mit Wasser Emulsionen jeder gewünschten Konzentration hergestellt werden.

| F3 Stäubemittel | a) | b) | c) | d) |
|---|---|---|---|---|
| Herbizid HE-4 | 2 % | 4 % | 2 % | 4 % |
| Safener S-1 | 3 % | 4 % | 4 % | 8 % |
| Talkum | 95 % | - | 94 % | - |
| Kaolin | - | 92 % | - | 88 % |

Man erhält anwendungsfertige Stäubemittel, indem das Wirkstoffgemisch mit dem Träger vermischt und auf einer geeigneten Mühle vermahlen wird.

| F4 Extruder Granulat | a) | b) | c) |
|---|---|---|---|
| Herbizid HE-4 | 5 % | 3 % | 5 % |
| Safener S-1 | 5 % | 7 % | 15 % |
| Na-Ligninsulfonat | 2 % | 2 % | 2 % |
| Carboxymethylcellulose | 1 % | 1 % | 1 % |
| Kaolin | 87 % | 87 % | 77 % |

Das Wirkstoffgemisch wird mit den Zusatzstoffen vermischt, vermahlen und mit Wasser angefeuchtet. Dieses Gemisch wird extrudiert und anschliessend im Luftstrom getrocknet.

| F5 Umhüllungs-Granulat | a) | b) |
|---|---|---|
| Herbizid HE-4 | 1,5 % | 3 % |
| Safener S-1 | 1,5 % | 5 % |
| Polyäthylenglykol (MG 200) | 3 % | 3 % |
| Kaolin | 94 % | 89 % |

Das fein gemahlene Wirkstoffgemisch wird in einem Mischer auf das mit Polyäthylenglykol angefeuchtete Kaolin gleichmässig aufgetragen. Auf diese Weise erhält man staubfreie Umhüllungs-Granulate.

| F6 Suspensions-Konzentrat | a) | b) |
|---|---|---|
| Herbizid HE-4 | 20 % | 20 % |
| Safener S-1 | 20 % | 40 % |
| Aethylenglykol | 10 % | 10 % |
| Nonylphenolpolyäthylenglykoläther (15 Mol AeO | 6 % | 6 % |
| Na-Ligninsulfonat | 10 % | 10 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 12 % |

Das fein gemahlene Wirkstoffgemisch wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Formulierungsbeispiele für Antidots der Formel I
(% = Gewichtsprozent)

| Fa-1 Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Fa-2 Emulsions-Konzentrate | |
|---|---|
| Wirkstoff aus Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Fa-3 Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| Fa-4 Extruder-Granulate | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| Fa-5 Umhüllungs-Granulate | |
|---|---|
| Wirkstoff aus Tabelle 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| Fa-6 Suspensions-Konzentrate | |
|---|---|
| Wirkstoff aus Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Patentansprüche

**1.** Die Verwendung von Antidots der Formel I

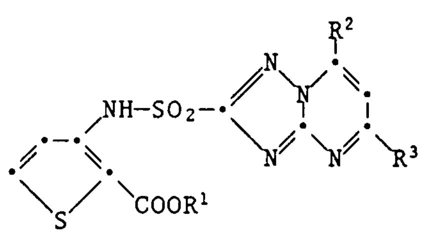

(I),

worin

$R^1$ $C_1$-$C_6$-Alkyl oder $C_3$-$C_6$-Cycloalkyl und

$R^2$ und $R^3$ unabhängig voneinander Wasserstoff, $C_1$-$C_3$-Alkyl, Trifluormethyl oder Cyclopropyl bedeuten,

mit der Massgabe, dass nur eines der Symbole $R^2$ und $R^3$ für Wasserstoff stehen kann, zum Schützen von Kulturpflanzen gegen die schädigende Wirkung von Herbiziden.

**2.** Verwendung gemäss Anspruch 1, dadurch gekennzeichnet, dass man ein Antidot der Formel I verwendet, in welchem $R^2$ und $R^3$ unabhängig voneinander $C_1$-$C_3$-Alkyl oder Cyclopropyl bedeuten und $R^1$ die für Formel I angegebenen Bedeutungen hat.

**3.** Verwendung gemäss Anspruch 1, dadurch gekennzeichnet, dass man ein Antidot der Formel I verwendet, in welchem eines der Symbole $R^2$ und $R^3$ für Methyl und das andere für Cyclopropyl steht und $R^1$ die für Formel I angegebenen Bedeutungen hat.

**4.** Verwendung gemäss Anspruch 1, dadurch gekennzeichnet, dass man ein Antidot der Formel I verwendet, in welchem $R^1$ Methyl bedeutet und $R^2$ und $R^3$ die für Formel I angegebenen Bedeutungen haben.

**5.** Verwendung gemäss Anspruch 1, dadurch gekennzeichnet, dass man als Antidot der Formel I N-(5,7-Dimethyl-1,2,4-triazolo[1,5-a]pyrimidin-2-ylsulfonyl)-3-aminothiophen-2-carbonsäuremethylester der Formel Ia

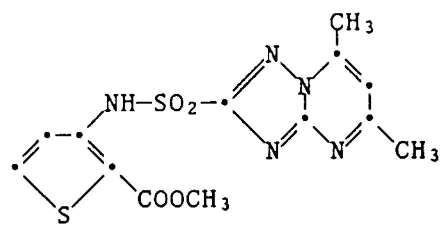

(Ia)

verwendet.

**6.** Verwendung gemäss Anspruch 1 von Antidots der Formel I gegen die schädigende Wirkung von herbiziden Sulfonylharnstoff-Derivaten der Formel II

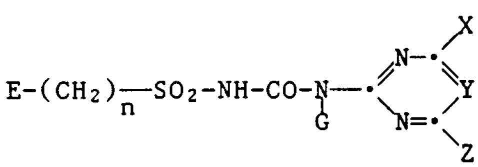

(II)

worin

E eines der Strukturelemente

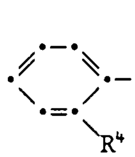 , 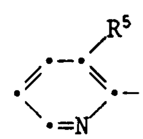 , 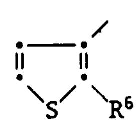 oder 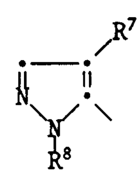

n die Zahl null oder eins,

G Wasserstoff oder Methyl,

X Methoxy, Aethoxy, Difluormethoxy, Methyl oder Chlor

Y CH oder N,

Z Methoxy, Methyl, Difluormethoxy, Cyclopropyl oder Methylamino,
$R^4$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R^5$ Trifluormethyl oder Di($C_1$-$C_4$-alkyl)carbamoyl,
$R^6$ $C_1$-$C_4$-Alkoxycarbonyl,
$R^7$ $C_1$-$C_4$-Alkoxycarbonyl, und
$R^8$ $C_1$-$C_4$-Alkyl bedeuten.

**7.** Verwendung gemäss Anspruch 1 von Antidots der Formel I gegen die schädigende Wirkung von herbiziden Chloracetaniliden der Formel III

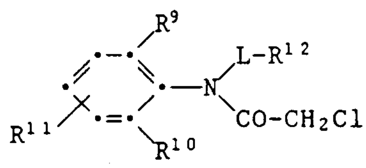

(III)

worin
L eine $C_1$-$C_5$-Alkylenbrücke,
$R^9$, $R^{10}$ und $R^{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R^{12}$ Wasserstoff, $C_1$-$C_4$-Alkoxy, -COOH, $C_1$-$C_4$-Alkoxycarbonyl, -$CONH_2$, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes 1,2,4-Triazol-3-yl,
gegebenenfalls substituiertes Dioxolanyl,
gegebenenfalls substituiertes Dioxanyl oder
gegebenenfalls substituiertes Tetrahydrofuryl bedeutet, oder das Strukturelement -L-$R^{12}$ für eine durch zwei $C_1$-$C_3$-Alkoxygruppen substituierte $C_1$-$C_4$-Alkylenbrücke oder für 5-Methyl-1,3,4-oxadiazol-2-yl steht.

**8.** Verwendung gemäss Anspruch 1 von Antidots der Formel I gegen die schädigende Wirkung von herbiziden Aryloxyphenoxypropionsäure-Derivaten der Formel IV

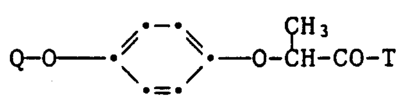

(IV)

worin
Q für den Rest

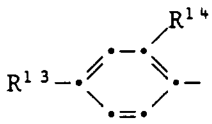 , 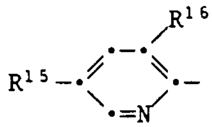 , 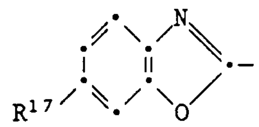 ,

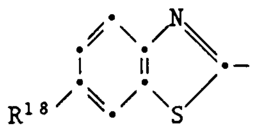 oder 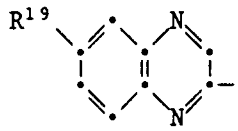

und T für

$-NR^{20}R^{21}$, $-N(CN)R^{22}$, $-OR^{23}$, $SR^{24}$ oder $-O-N=CR^{25}R^{26}$ stehen, wobei

| | |
|---|---|
| $R^{13}$ und $R^{15}$ | Halogen oder Trifluormethyl, |
| $R^{14}$, $R^{16}$, $R^{17}$, $R^{18}$ und $R^{19}$ | Wasserstoff oder Halogen, |
| $R^{20}$ und $R^{21}$ | unabhängig voneinander Wasserstoff, $C_1$-$C_8$-Alkoxy, $C_1$-$C_8$-Alkyl, Phenyl oder Benzyl oder $R^{20}$ und $R^{21}$ zusammen mit dem sie tragenden Stickstoffatom einen 5- bis 6-gliedrigen gesättigten Stickstoffheterocyclus, der durch ein Sauerstoff-oder Schwefelatom unterbrochen sein kann, |
| $R^{22}$ | $C_1$-$C_4$-Alkyl, $C_3$-$C_4$-Alkenyl, $C_3$-$C_4$-Alkinyl oder $C_2$-$C_4$-Alkoxyalkyl |
| $R^{23}$ | Wasserstoff oder Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein-oder mehrfach durch Amino, Halogen, Hydroxyl, Cyan, Nitro, Phenyl, $C_1$-$C_4$-Alkoxy, Polyethoxy mit 2 bis 6 Ethylenoxideinheiten, - $COOR^{27}$, $-COSR^{28}$, $-CONH_2$, -CON($C_1$-$C_4$-alkoxy)-$C_1$-$C_4$-alkyl, -CO-N-di-$C_1$-$C_4$-alkyl, CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, $-COOR^{29}$, $COSR^{30}$, - $CONH_2$, -CON-($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl, |
| $R^{25}$ und $R^{26}$ | unabhängig voneinander $C_1$-$C_4$-Alkyl oder zusammen eine 3-bis 6-gliedrige Alkylenkette und |
| $R^{27}$, $R^{28}$, $R^{29}$ und $R^{30}$ | unabhängig voneinander Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkyl, $C_3$-$C_6$-Alkinyl oder $C_3$-$C_6$-Halogenalkinyl bedeuten. |

**9.** Verwendung gemäss Anspruch 1 von Antidots der Formel I gegen die schädigende Wirkung von herbiziden N-Benzoyl-N-phenylalanin-Derivaten der Formel V

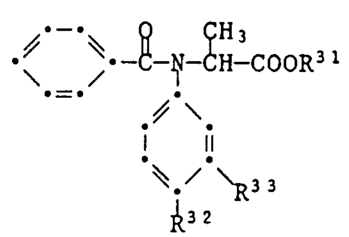 (V),

worin

| | |
|---|---|
| $R^{31}$ | Wasserstoff oder $C_1$-$C_4$-Alkyl und |
| $R^{32}$ und $R^{33}$ | unabhängig voneinander Fluor oder Chlor bedeuten. |

**10.** Verfahren zum Schützen von Kulturpflanzen gegen die kulturpflanzenschädigende Wirkung von Herbiziden, dadurch gekennzeichnet, dass man vor, während oder nach der Applikation des Herbizids die Kulturpflanze oder deren Lebensraum oder Samen oder Stecklinge der Kulturpflanze mit einer antagonistisch wirksamen Menge einer Verbindung der Formel I

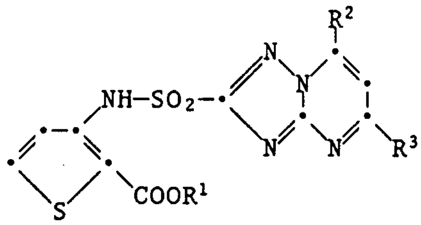

(I),

worin

| | |
|---|---|
| $R^1$ | $C_1$-$C_6$-Alkyl oder $C_3$-$C_6$-Cycloalkyl und |
| $R^2$ und $R^3$ | unabhängig voneinander Wasserstoff, $C_1$-$C_3$-Alkyl, Trifluormethyl oder Cyclopropyl bedeuten, |

mit der Massgabe, dass nur eines der Symbole $R^2$ und $R^3$ für Wasserstoff stehen kann, behandelt.

**11.** Herbizides Mittel, dadurch gekennzeichnet, dass es ein Herbizid und eine die kulturpflanzenschädigende Wirkung dieses Herbizids antagonisierende Menge einer Verbindung der Formel I

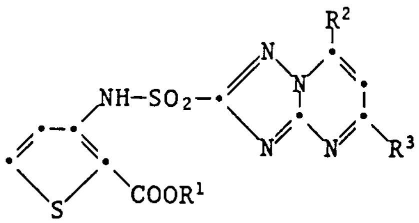

(I),

worin

| | |
|---|---|
| $R^1$ | $C_1$-$C_6$-Alkyl oder $C_3$-$C_6$-Cycloalkyl und |
| $R^2$ und $R^3$ | unabhängig voneinander Wasserstoff, $C_1$-$C_3$-Alkyl, Trifluormethyl oder Cyclopropyl bedeuten, |

mit der Massgabe, dass nur eines der Symbole $R^2$ und $R^3$ für Wasserstoff stehen kann,
sowie gewünschtenfalls Hilfsstoffe, Trägerstoffe oder Hilfs- und Trägerstoffe enthält.

**12.** Herbizides Mittel gemäss Anspruch 11, dadurch gekennzeichnet, dass es als Herbizid ein Sulfonylharnstoffderivat der Formel II, wie im Anspruch 6 beschrieben, enthält.

**13.** Herbizides Mittel gemäss Anspruch 11, dadurch gekennzeichnet, dass es als Herbizid ein Chloracetanilid der Formel III, wie im Anspruch 7 beschrieben, enthält.

**14.** Herbizides Mittel gemäss Anspruch 11, dadurch gekennzeichnet, dass es als Herbizid ein Aryloxyphenoxypropionsäurederivat der Formel IV, wie im Anspruch 8 beschrieben, enthält.

**15.** Herbizides Mittel gemäss Anspruch 11, dadurch gekennzeichnet, dass es als Herbizid ein N-Benzoyl-N-phenylalanin der Formel V, wie im Anspruch 9 beschrieben, enthält.

**16.** Mittel zum Schützen von Kulturpflanzen gegen die schädigende Wirkung von Herbiziden, dadurch gekennzeichnet, dass es aus mindestens einer Verbindung der Formel I'

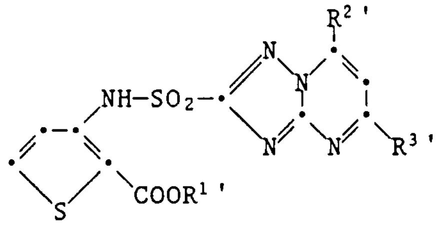

(I'),

worin

| | |
|---|---|
| $R^{1'}$ | $C_1$-$C_6$-Alkyl oder $C_3$-$C_6$-Cycloalkyl und |
| $R^{2'}$ und $R^{3'}$ | unabhängig voneinander Wasserstoff, $C_1$-$C_3$-Alkyl, Trifluormethyl oder Cyclopropyl bedeuten, |

mit der Massgabe, dass mindestens eines der Symbole $R^{2'}$ und $R^{3'}$ für Cyclopropyl steht, und ein oder mehreren Hilfsstoffen, Trägerstoffen oder Hilfs- und Trägerstoffen besteht.

**17.** Verbindungen der Formel I'

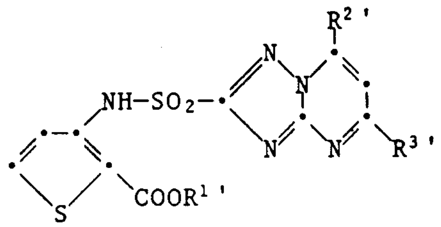

(I'),

worin

| | |
|---|---|
| $R^{1'}$ | $C_1$-$C_6$-Alkyl oder $C_3$-$C_6$-Cycloalkyl und |
| $R^{2'}$ und $R^{3'}$ | unabhängig voneinander Wasserstoff, $C_1$-$C_3$-Alkyl, Trifluormethyl oder Cyclopropyl bedeuten, |

mit der Massgabe, dass mindestens eines der Symbole $R^{2'}$ und $R^{3'}$ für Cyclopropyl steht.

**18.** Verbindungen der Formel I' gemäss Anspruch 17, dadurch gekennzeichnet, dass eines der Symbole $R^{2'}$ und $R^{3'}$ für Cyclopropyl und das andere für Methyl steht und $R^{1'}$ die für Formel I' angegebenen Bedeutungen hat.

**19.** Verbindungen der Formel I' gemäss Anspruch 17, dadurch gekennzeichnet, dass $R^{1'}$ für Methyl steht und $R^{2'}$ und $R^{3'}$ die für Formel I' angegebenen Bedeutungen haben.

**20.** Verbindungen der Formel I' gemäss Anspruch 17, dadurch gekennzeichnet, dass $R^{1'}$ für Methyl steht und eines der Symbole $R^{2'}$ und $R^{3'}$ für Cyclopropyl und das andere für Methyl steht.

**21.** Verbindungen der Formel I' gemäss Anspruch 17, dadurch gekennzeichnet, dass $R^{1'}$ für $C_1$-$C_3$-Alkyl steht und $R^{2'}$ und $R^{3'}$ unabhängig voneinander Wasserstoff, Methyl oder Cyclopropyl bedeuten, mit der Massgabe, dass nur eines der Symbole $R^{2'}$ und $R^{3'}$ für Wasserstoff stehen kann.

**22.** Verfahren zur Herstellung von Verbindungen der Formel I'

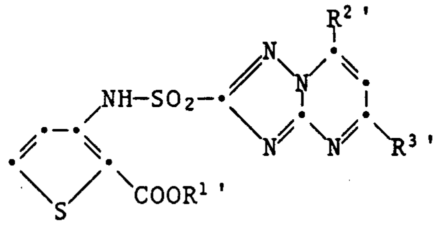

(I'),

worin

$R^{1'}$ $C_1$-$C_6$-Alkyl oder $C_3$-$C_6$-Cycloalkyl und

$R^{2'}$ und $R^{3'}$ unabhängig voneinander Wasserstoff, $C_1$-$C_3$-Alkyl, Trifluormethyl oder Cyclopropyl bedeuten,

mit der Massgabe, dass mindestens eines der Symbole $R^{2'}$ und $R^{3'}$ für Cyclopropyl steht, dadurch gekennzeichnet, dass man eine Verbindung der Formel VI

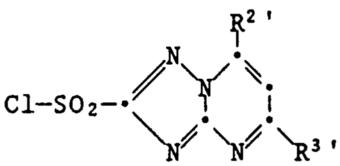

(VI),

worin $R^{2'}$ und $R^{3'}$ unabhängig voneinander Wasserstoff, $C_1$-$C_3$-Alkyl, Trifluormethyl oder Cyclopropyl bedeuten, mit der Massgabe, dass mindestens eines der Symbole $R^{2'}$ und $R^{3'}$ für Cyclopropyl steht, mit einer Verbindung der Formel VII

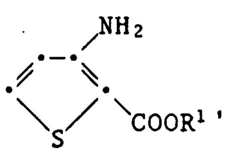

(VII),

worin $R^{1'}$ $C_1$-$C_6$-Alkyl oder $C_3$-$C_6$-Cycloalkyl bedeutet, umsetzt.

**23.** Verfahren zur Herstellung von Verbindungen der Formel VI

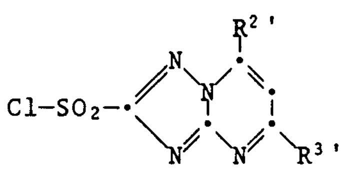

(VI),

worin $R^{2'}$ und $R^{3'}$ unabhängig voneinander Wasserstoff, $C_1$-$C_3$-Alkyl, Trifluormethyl oder Cyclopropyl bedeuten, mit der Massgabe, dass mindestens eines der Symbole $R^{2'}$ und $R^{3'}$ für Cyclopropyl steht, dadurch gekennzeichnet, dass man eine Verbindung der Formel VIII

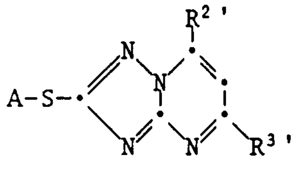

(VIII),

worin $R^{2'}$ und $R^{3'}$ unabhängig voneinander Wasserstoff, $C_1$-$C_3$-Alkyl, Trifluormethyl oder Cyclopropyl bedeuten, mit der Massgabe, dass mindestens eines der Symbole $R^{2'}$ und $R^{3'}$ für Cyclopropyl steht und A Wasserstoff oder Benzyl bedeutet, in wässrigem, saurem Medium mit Chlor behandelt.

**24.** Verfahren zur Herstellung von Verbindungen der Formel VIII

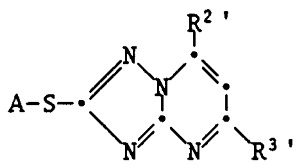

(VIII),

worin $R^{2'}$ und $R^{3'}$ unabhängig voneinander Wasserstoff, $C_1$-$C_3$-Alkyl, Trifluormethyl oder Cyclopropyl bedeuten, mit der Massgabe, dass mindestens eines der Symbole $R^{2'}$ und $R^{3'}$ für Cyclopropyl steht und A Wasserstoff oder Benzyl bedeutet, dadurch gekennzeichnet, dass man eine Verbindung der Formel IX

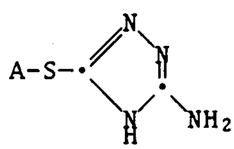

(IX),

worin A Wasserstoff oder Benzyl bedeutet, mit einer Verbindung der Formel X

$R_b$-CO-$CH_2$-$R_a$ (X),

worin i) $R_a$ für die Gruppe -CO-$R_c$ steht und eines der Symbole $R_b$ und $R_c$ für $R^{2'}$ und das andere für $R^{3'}$ steht oder ii) $R_a$ für die Gruppe -CH($OR_d$)$_2$ steht und $R_b$ für $R^{2'}$ oder $R^{3'}$ und $R_d$ für Methyl oder Aethyl steht, umsetzt.

**25.** Verbindungen der Formel VI

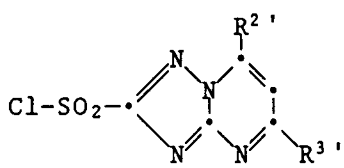

(VI),

worin $R^{2'}$ und $R^{3'}$ unabhängig voneinander Wasserstoff, $C_1$-$C_3$-Alkyl, Trifluormethyl oder Cyclopropyl bedeuten, mit der Massgabe, dass mindestens eines der Symbole $R^{2'}$ und $R^{3'}$ für Cyclopropyl steht.

**26.** Verbindungen der Formel VIII

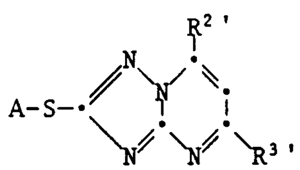

(VIII),

worin $R^{2'}$ und $R^{3'}$ unabhängig voneinander Wasserstoff, $C_1$-$C_3$-Alkyl, Trifluormethyl oder Cyclopropyl bedeuten, mit der Massgabe, dass mindestens eines der Symbole $R^{2'}$ und $R^{3'}$ für Cyclopropyl steht und A Wasserstoff oder Benzyl bedeutet.

**27.** Saatgut von Kulturpflanzen, welches mit einer antagonistisch wirksamen Menge einer Verbindung der Formel I

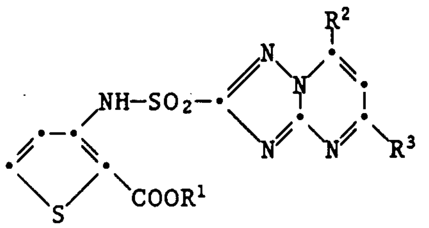

(I),

worin $R^1$ $C_1$-$C_6$-Alkyl oder $C_3$-$C_6$-Cycloalkyl und $R^2$ und $R^3$ unabhängig voneinander Wasserstoff, $C_1$-$C_3$-Alkyl, Trifluormethyl oder Cyclopropyl bedeuten, mit der Massgabe, dass nur eines der Symbole $R^2$ und $R^3$ für Wasserstoff stehen kann, behandelt worden ist.

**28.** Verfahren zur selektiven Bekämpfung von Unkräutern in Kulturpflanzenbeständen, dadurch gekennzeichnet, dass man die Kulturpflanzenbestände, Teile der Kulturpflanzen oder deren Anbauflächen mit einem Herbizid und einer Verbindung der Formel I

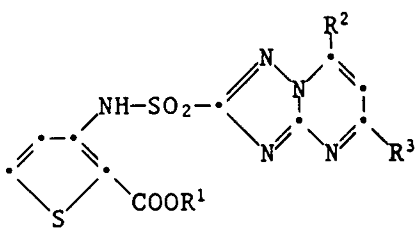

(I),

worin $R^1$ $C_1$-$C_6$-Alkyl oder $C_3$-$C_6$-Cycloalkyl und $R^2$ und $R^3$ unabhängig voneinander Wasserstoff, $C_1$-$C_3$-Alkyl, Trifluormethyl oder Cyclopropyl bedeuten, mit der Massgabe, dass nur eines der Symbole $R^2$ und $R^3$ für Wasserstoff stehen kann, behandelt.

## Claims

**1.** The use of an antidote of formula I

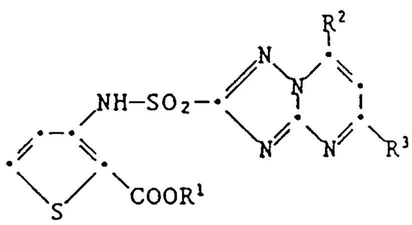

(I)

wherein

| | |
|---|---|
| $R^1$ | is $C_1$-$C_6$alkyl or $C_3$-$C_6$cycloalkyl, and |
| each of $R^2$ and $R^3$, | independently of the other, is hydrogen, $C_1$-$C_3$alkyl, trifluoromethyl or cyclopropyl, |

with the proviso that only one of the symbols $R^2$ and $R^3$ may be hydrogen,
for protecting cultivated plants from the harmful effect of herbicides.

**2.** The use according to claim 1, wherein an antidote of formula I wherein each of $R^2$ and $R^3$, independently of the other, is $C_1$-$C_3$alkyl or cyclopropyl, and $R^1$ is as defined for formula I is used.

**3.** The use according to claim 1, wherein an antidote of formula I wherein one of the symbols $R^2$ and $R^3$ is methyl and the other is cyclopropyl, and $R^1$ is as defined for formula I is used.

**4.** The use according to claim 1, wherein an antidote of formula I wherein $R^1$ is methyl and $R^2$ and $R^3$ are as defined for formula I is used.

**5.** The use according to claim 1, wherein N-(5,7-dimethyl-1,2,4-triazolo[1,5-a]pyrimidin-2-ylsulfonyl)-3-aminothiophene-2-carboxylic acid methyl ester of formula Ia

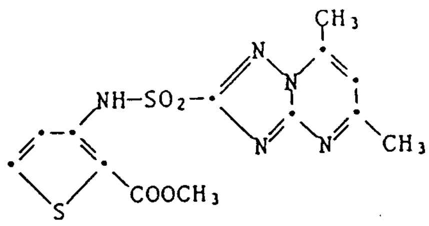

(Ia)

is used as the antidote of formula I.

**6.** The use according to claim 1 of an antidote of formula I against the harmful effect of herbicidal sulfonylurea derivatives of formula II

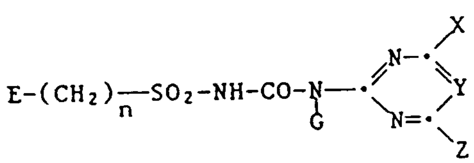

(II)

wherein

E is one of the structural elements

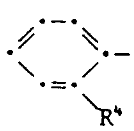 , 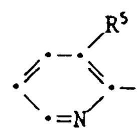 , 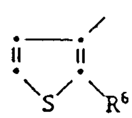 or 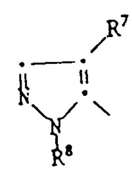

n is the number 0 or 1,
G is hydrogen or methyl,
X is methoxy, ethoxy, difluoromethoxy, methyl or chlorine,
Y is CH or N,
Z is methoxy, methyl, difluoromethoxy, cyclopropyl or methylamino,
$R^4$ is $C_2$-$C_5$alkoxyalkoxy, $C_1$-$C_4$haloalkoxy, $C_1$-$C_4$haloalkylthio, $C_2$-$C_4$haloalkenyl, chlorine or $C_1$-$C_4$alkoxycarbonyl,
$R^5$ is trifluoromethyl or di($C_1$-$C_4$alkyl)carbamoyl,
$R^6$ is $C_1$-$C_4$alkoxycarbonyl,
$R^7$ is $C_1$-$C_4$alkoxycarbonyl, and
$R^8$ is $C_1$-$C_4$alkyl.

**7.** The use according to claim 1 of an antidote of formula I against the harmful effect of herbicidal chloroacetanilides of formula III

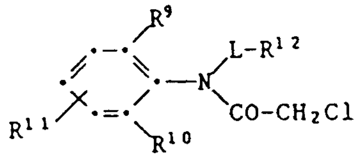

(III)

wherein

L is a $C_1$-$C_5$alkylene bridge,

each of $R^9$, $R^{10}$ and $R^{11}$, independently of the others, is hydrogen, halogen, $C_1$-$C_4$alkyl, $C_1$-$C_4$alkoxy, $C_1$-$C_4$haloalkyl, $C_2$-$C_5$alkoxyalkyl or $C_2$-$C_5$alkylthioalkyl, and

$R^{12}$ is hydrogen, $C_1$-$C_4$alkoxy, -COOH, $C_1$-$C_4$alkoxycarbonyl, $-CONH_2$, $C_1$-$C_4$alkylcarbamoyl, di-$C_1$-$C_4$alkylcarbamoyl, cyano, $C_1$-$C_4$alkylcarbonyl,
unsubstituted or substituted benzoyl,
unsubstituted or substituted furyl,
unsubstituted or substituted thienyl,
unsubstituted or substituted pyrrolyl,
unsubstituted or substituted pyrazolyl,
unsubstituted or substituted 1,3,4-oxadiazol-2-yl,
unsubstituted or substituted 1,3,4-thiadiazol-2-yl,
unsubstituted or substituted 1,2,4-triazol-3-yl,
unsubstituted or substituted dioxolanyl,
unsubstituted or substituted dioxanyl or
unsubstituted or substituted tetrahydrofuryl, or the structural element -L-$R^{12}$ is a $C_1$-$C_4$alkylene bridge substituted by two $C_1$-$C_3$alkoxy groups or is 5-methyl-1,3,4-oxadiazol-2-yl.

**8.** The use according to claim 1 of an antidote of formula I against the harmful effect of herbicidal aryloxyphenoxypropionic acid derivatives of formula IV

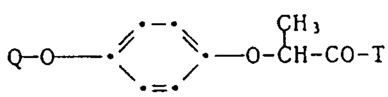

(IV)

wherein Q is a radical

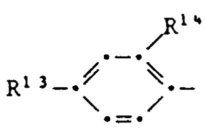 , 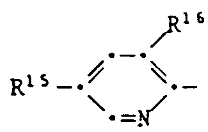 , 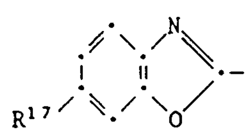 ,

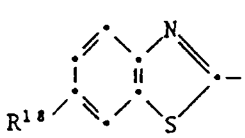 or 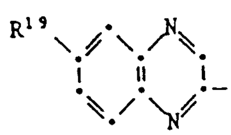

and T is $-NR^{20}R^{21}$, $-N(CN)R^{22}$, $-OR^{23}$, $SR^{24}$ or $-O-N=CR^{25}R^{26}$, wherein

| | |
|---|---|
| $R^{13}$ and $R^{15}$ | are halogen or trifluoromethyl, |
| $R^{14}$, $R^{16}$, $R^{17}$, $R^{18}$ and $R^{19}$ | are hydrogen or halogen, |
| each of $R^{20}$ and $R^{21}$, | independently of the other, is hydrogen, $C_1$-$C_8$alkoxy, $C_1$-$C_8$alkyl, phenyl or benzyl, or $R^{20}$ and $R^{21}$ together with the nitrogen atom carrying them form a 5- or 6-membered saturated nitrogen heterocycle that may be interrupted by an oxygen or a sulfur atom, |
| $R^{22}$ | is $C_1$-$C_4$alkyl, $C_3$-$C_4$alkenyl, $C_3$-$C_4$alkynyl or $C_2$-$C_4$alkoxyalkyl, |

$R^{23}$ is hydrogen or the equivalent of an alkali metal, alkaline earth metal, copper or iron ion; a quaternary $C_1$-$C_4$alkylammonium or $C_1$-$C_4$hydroxyalkylammonium radical; a $C_1$-$C_9$alkyl radical which is unsubstituted or mono- or poly-substituted by amino, halogen, hydroxy, cyano, nitro, phenyl, $C_1$-$C_4$alkoxy, polyethoxy having from 2 to 6 ethylene oxide units, -COOR$^{27}$, -COSR$^{28}$, -CONH$_2$, CON($C_1$-$C_4$alkoxy)-$C_1$-$C_4$-alkyl, -CO-N-di-$C_1$-$C_4$alkyl, CONH-$C_1$-$C_4$alkyl, -N($C_1$-$C_4$alkoxy)-$C_1$-$C_4$alkyl or by di-$C_1$-$C_4$alkylamino; a $C_3$-$C_9$alkenyl radical which is unsubstituted or substituted by halogen or by $C_1$-$C_4$alkoxy; a $C_3$-$C_9$-alkynyl radical which is unsubstituted or substituted by halogen or by $C_1$-$C_4$alkoxy; $C_3$-$C_9$cycloalkyl; or phenyl which is unsubstituted or substituted by cyano, $C_1$-$C_4$alkyl, $C_1$-$C_4$alkoxy, acetyl, -COOR$^{29}$, COSR$^{30}$, -CONH$_2$, -CON($C_1$-$C_4$alkoxy)-$C_1$-$C_4$-alkyl, -CO-N-di-$C_1$-$C_4$alkyl or by -CONH-$C_1$ $C_4$alkyl,

each of $R^{25}$ and $R^{26}$, independently of the other, is $C_1$-$C_4$alkyl, or $R^{25}$ and $R^{26}$ together form a 3-to 6-membered alkylene chain, and

each of $R^{27}$, $R^{28}$, $R^{29}$ and $R^{30}$, independently of the others, is hydrogen, $C_1$-$C_6$alkyl, $C_1$-$C_6$haloalkyl, $C_2$-$C_6$alkoxyalkyl, $C_3$-$C_6$alkenyl, $C_3$-$C_6$haloalkyl, $C_3$-$C_6$alkynyl or $C_3$-$C_6$haloalkynyl.

**9.** The use according to claim 1 of an antidote of formula I against the harmful effect of herbicidal N-benzoyl-N-phenylalanine derivatives of formula V

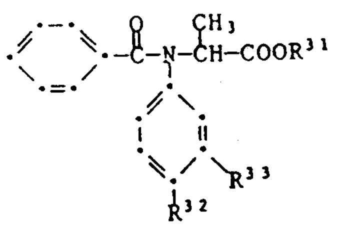

(V)

wherein
$R^{31}$ is hydrogen or $C_1$-$C_4$alkyl and
each of $R^{32}$ and $R^{33}$, independently of the other, is fluorine or chlorine.

**10.** A method of protecting cultivated plants from the harmful effect of herbicides, which comprises treating the cultivated plant or the locus thereof or seeds or cuttings of the cultivated plant, before, during or after application of the herbicide, with an antagonistically effective amount of a compound of formula I

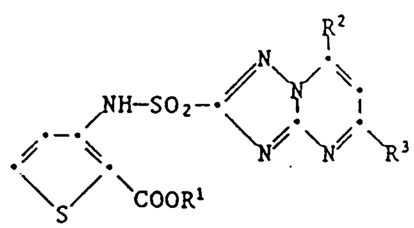

(I)

wherein
$R^1$ is $C_1$-$C_6$alkyl or $C_3$-$C_6$cycloalkyl, and
each of $R^2$ and $R^3$, independently of the other, is hydrogen, $C_1$-$C_3$alkyl, trifluoromethyl or cyclopropyl,
with the proviso that only one of the symbols $R^2$ and $R^3$ may be hydrogen.

**11.** A herbicidal composition which comprises a herbicide and a compound of formula I

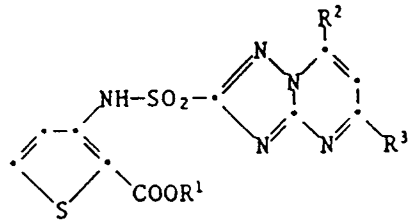

(I),

wherein

| | |
|---|---|
| $R^1$ | is $C_1$-$C_6$alkyl or $C_3$-$C_6$cycloalkyl, and |
| each of $R^2$ and $R^3$, | independently of the other, is hydrogen, $C_1$-$C_3$alkyl, trifluoromethyl or cyclopropyl, |

with the proviso that only one of the symbols $R^2$ and $R^3$ may be hydrogen,
in an amount that antagonises the harmful effect of that herbicide on the cultivated plant, and, if desired, adjuvants, carriers or adjuvants and carriers.

**12.** A herbicidal composition according to claim 11, which comprises as herbicide a sulfonylurea derivative of formula II as described in claim 6.

**13.** A herbicidal composition according to claim 11, which comprises as herbicide a chloroacetanilide of formula III as described in claim 7.

**14.** A herbicidal composition according to claim 11, which comprises as herbicide an aryloxyphenoxypropionic acid derivative of formula IV as described in claim 8.

**15.** A herbicidal composition according to claim 11, which comprises as herbicide an N-benzoyl-N-phenylalanine of formula V as described in claim 9.

**16.** A composition for protecting cultivated plants from the harmful effect of herbicides, which consists of at least one compound of formula I'

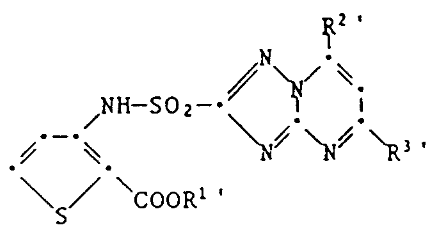

(I')

wherein

| | |
|---|---|
| $R^{1'}$ | is $C_1$-$C_6$alkyl or $C_3$-$C_6$cycloalkyl, and |
| each of $R^{2'}$ and $R^{3'}$, | independently of the other, is hydrogen, $C_1$-$C_3$alkyl, trifluoromethyl or cyclopropyl, |

with the proviso that at least one of the symbols $R^{2'}$ and $R^{3'}$ is cyclopropyl,
and one or more adjuvants, carriers or adjuvants and carriers.

**17.** A compound of formula I'

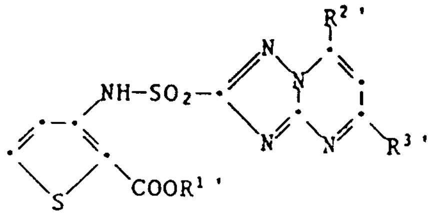

(I')

wherein

$R^{1'}$ is $C_1$-$C_6$alkyl or $C_3$-$C_6$cycloalkyl, and

each of $R^{2'}$ and $R^{3'}$, independently of the other, is hydrogen, $C_1$-$C_3$alkyl, trifluoromethyl or cyclopropyl,

with the proviso that at least one of the symbols $R^{2'}$ and $R^{3'}$ is cyclopropyl.

**18.** A compound of formula I' according to claim 17, wherein one of the symbols $R^{2'}$ and $R^{3'}$ is cyclopropyl and the other is methyl, and $R^{1'}$ is as defined for formula I'.

**19.** A compound of formula I' according to claim 17, wherein $R^{1'}$ is methyl and $R^{2'}$ and $R^{3'}$ are as defined for formula I'.

**20.** A compound of formula I' according to claim 17, wherein $R^{1'}$ is methyl and one of the symbols $R^{2'}$ and $R^{3'}$ is cyclopropyl and the other is methyl.

**21.** A compound of formula I' according to claim 17, wherein $R^{1'}$ is $C_1$-$C_3$alkyl, and each of $R^{2'}$ and $R^{3'}$, independently of the other, is hydrogen, methyl or cyclopropyl, with the proviso that only one of the symbols $R^{2'}$ and $R^{3'}$ may be hydrogen.

**22.** A process for the preparation of a compound of formula I'

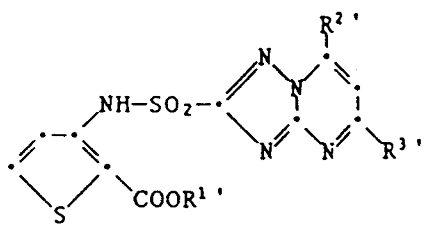

(I')

wherein

$R^{1'}$ is $C_1$-$C_6$alkyl or $C_3$-$C_6$cycloalkyl, and

each of $R^{2'}$ and $R^{3'}$, independently of the other, is hydrogen, $C_1$-$C_3$alkyl, trifluoromethyl or cyclopropyl,

with the proviso that at least one of the symbols $R^{2'}$ and $R^{3'}$ is cyclopropyl, which comprises reacting a compound of formula VI

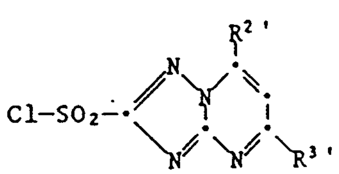

(VI),

wherein each of $R^{2'}$ and $R^{3'}$, independently of the other, is hydrogen, $C_1$-$C_3$alkyl, trifluoromethyl or

cyclopropyl, with the proviso that at least one of the symbols $R^{2'}$ and $R^{3'}$ is cyclopropyl, with a compound of formula VII

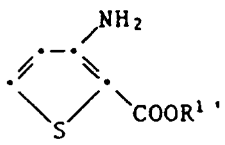

(VII),

wherein $R^{1'}$ is $C_1$-$C_6$alkyl or $C_3$-$C_6$cycloalkyl.

**23.** A process for the preparation of a compound of formula VI

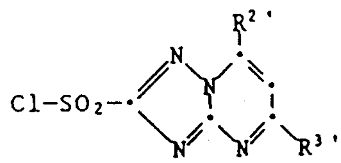

(VI),

wherein each of $R^{2'}$ and $R^{3'}$, independently of the other, is hydrogen, $C_1$-$C_3$alkyl, trifluoromethyl or cyclopropyl, with the proviso that at least one of the symbols $R^{2'}$ and $R^{3'}$ is cyclopropyl, which comprises treating a compound of formula VIII

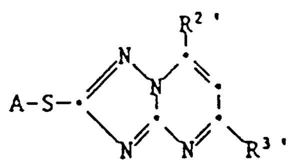

(VIII),

wherein each of $R^{2'}$ and $R^{3'}$, independently of the other, is hydrogen, $C_1$-$C_3$alkyl, trifluoromethyl or cyclopropyl, with the proviso that at least one of the symbols $R^{2'}$ and $R^{3'}$ is cyclopropyl, and A is hydrogen or benzyl, with chlorine in an aqueous acidic medium.

**24.** A process for the preparation of a compound of formula VIII

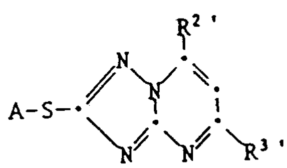

(VIII),

wherein each of $R^{2'}$ and $R^{3'}$, independently of the other, is hydrogen, $C_1$-$C_3$alkyl, trifluoromethyl or cyclopropyl, with the proviso that at least one of the symbols $R^{2'}$ and $R^{3'}$ is cyclopropyl, and A is hydrogen or benzyl, which comprises reacting a compound of formula IX

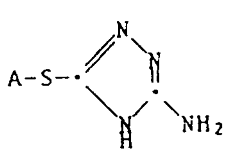

(IX),

wherein A is hydrogen or benzyl, with a compound of formula X

$R_b$-CO-$CH_2R_a$ (X),

wherein i) $R_a$ is a group -CO-$R_c$ and one of the symbols $R_b$ and $R_c$ is $R^{2'}$ and the other is $R^{3'}$, or ii) $R_a$ is a group -CH($OR_d$)$_2$ and $R_b$ is $R^{2'}$ or $R^{3'}$ and $R_d$ is methyl or ethyl.

**25.** A compound of formula VI

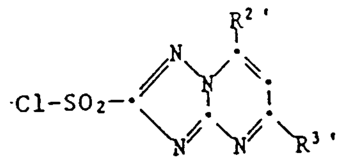

(VI)

wherein each of $R^{2'}$ and $R^{3'}$, independently of the other, is hydrogen, $C_1$-$C_3$alkyl, trifluoromethyl or cyclopropyl, with the proviso that at least one of the symbols $R^{2'}$ and $R^{3'}$ is cyclopropyl.

**26.** A compound of formula VIII

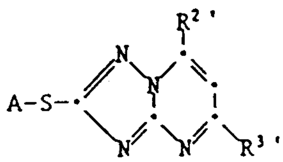

(VIII)

wherein each of $R^{2'}$ and $R^{3'}$, independently of the other, is hydrogen, $C_1$-$C_3$alkyl, trifluoromethyl or cyclopropyl, with the proviso that at least one of the symbols $R^{2'}$ and $R^{3'}$ is cyclopropyl, and A is hydrogen or benzyl.

**27.** Seeds of cultivated plants which have been treated with an antagonistically effective amount of a compound of formula I

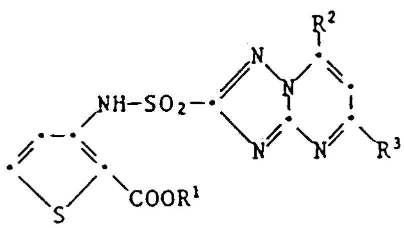

(I)

wherein $R^1$ is $C_1$-$C_6$alkyl or $C_3$-$C_6$cycloalkyl, and each of $R^2$ and $R^3$, independently of the other, is hydrogen, $C_1$-$C_3$alkyl, trifluoromethyl or cyclopropyl, with the proviso that only one of the symbols $R^2$ and $R^3$ may be hydrogen.

**28.** A method of selectively controlling weeds in crops of cultivated plants, which comprises treating the crops of cultivated plants, parts of the cultivated plants or the cultivation areas of the cultivated plants with a herbicide and a compound of formula I

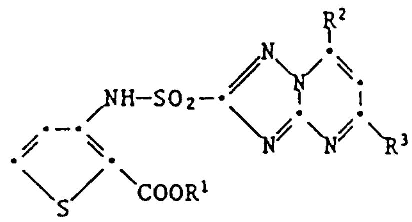

(I)

wherein $R^1$ is $C_1$-$C_6$alkyl or $C_3$-$C_6$cycloalkyl, and each of $R^2$ and $R^3$, independently of the other, is hydrogen, $C_1$-$C_3$alkyl, trifluoromethyl or cyclopropyl, with the proviso that only one of the symbols $R^2$ and $R^3$ may be hydrogen.

**Revendications**

1. L'utilisation d'antidotes de formule I

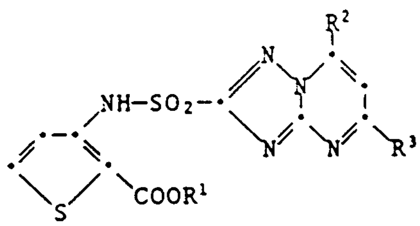

I

dans laquelle
R1 représente un groupe alkyle en C1-C6 ou cycloalkyle en C3-C6 et
R2 et R3 représentent chacun, indépendamment l'un de l'autre, l'hydrogène, un groupe alkyle en C1-C3, trifluorométhyle ou cyclopropyle,
sous réserve qu'un seul des symboles R2 et R3 peut représenter l'hydrogène, pour la protection des végétaux cultivés contre les dommages provoqués par les herbicides.

2. Utilisation selon revendication 1, caractérisée en ce que l'on utilise un antidote de formule I dans laquelle R2 et R3 représentent chacun, indépendamment l'un de l'autre, un groupe alkyle en C1-C3 ou cyclopropyle et R1 a les significations indiquées en référence à la formule I;

3. Utilisation selon revendication 1, caractérisée en ce que l'on utilise un antidote de formule I dans laquelle l'un des symboles R2 et R3 représente un groupe méthyle et l'autre un groupe cyclopropyle, et R1 a les significations indiquées en référence à la formule I,

4. Utilisation selon revendication 1, caractérisée en ce que l'on utilise un antidote de formule I dans laquelle R1 représente un groupe méthyle, et R2 et R3 ont les significations indiquées en référence à la formule I.

5. Utilisation selon revendication 1, caractérisée en ce que l'on utilise en tant qu'antidote de formule I le N-(5,7-diméthyl-1,2,4-triazolo-[1,5-a)-pyrimidine-2-ylsulfonyl-3-aminothiophène-2-carboxylate de méthyle de formule Ia

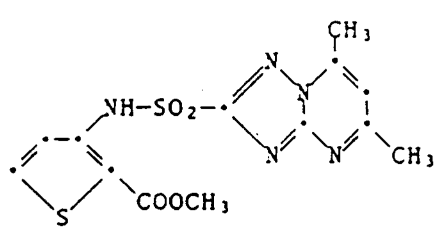

Ia

6. Utilisation selon revendication 1 d'antidotes de formule I pour la protection contre les dommages provoqués par des dérivés herbicides de sulfonylurées de formule II

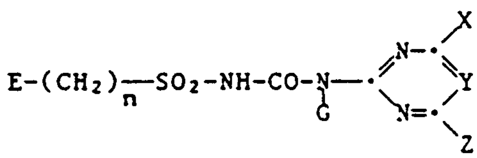

II

dans laquelle
E représente l'un des éléments de structure

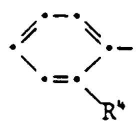 , 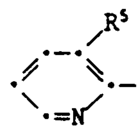 , 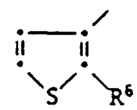 ou 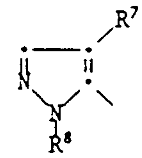

n est égal à O ou I,
G représente l'hydrogène ou un groupe méthyle,
X représente un groupe méthoxy, éthoxy, difluorométhoxy, méthyle ou le chlore,
Y représente CH ou N,
Z représente un groupe méthoxy, méthyle, difluorométhoxy, cyclopropyle ou méthylamino,
R4 représente un groupe alcoxy en C2-C5, halogénoalcoxy en C1-C4, halogénoalkythio en C1-C4, halogénoalcényle en C2-C4, le chlore ou un groupe (alcoxy en C1-C4)-carbonyle,
R5 représente un groupe trifluorométhyle ou di-(alkyle en C1-C4)-carbamoyle,
R6 représente un groupe (alcoxy en C1-C4)-carbonyle,
R7 représente un groupe (alcoxy en C1-C4)-carbonyle et
R8 représente un groupe alkyle en C1-C4.

7. Utilisation selon revendication 1 d'antidotes de formule I pour la protection contre les dommages provoqués par des chloracétanilides herbicides de formule III

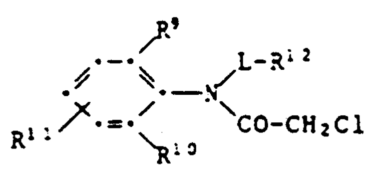

III

dans laquelle
L représente un pont alkylène en C1-C5,
R9, R1O et R11 représentent chacun, indépendamment les uns des autres, l'hydrogène, un halogène, un groupe alkyle en C1-C4, alcoxy en C1-C4, halogénoalkyle en C1-C4, alcoxyalkyle en C2-C5 ou alkylthioalkyle en C2-C5, R12 représente l'hydrogène, un groupe alcoxy en C1-C4, -COOH, (alcoxy en C1-C4)-carbonyle, -CONH2, (alkyle en C1-C4)-carbamoyle, di-(alkyle en C1-C4)-carbamoyle, cyano, (alkyle en C1-C4)-carbonyle,
benzoyle éventuellement substitué
furyle éventuellement substitué
thiényle éventuellement substitué
pyrrolyle éventuellement substitué
pyrazolyle éventuellement substitué
1,3,4-oxadiazole-2-yl éventuellement substitué
1,3,4-thiodiazole-2-yle éventuellement substitué

1,2,4-triazole-3-yle éventuellement substitué
dioxolannyle éventuellement substitué
dioxannyle éventuellement substitué
tétrahydrofuryle éventuellement substitué,

ou bien l'élément de structure L-R12 représente un pont alkylène en C1-C4 substitué par deux groupes alcoxy en C1-C3 ou un groupe 5-méthyl-1,3,4-oxadiazole-2-yle.

8. Utilisation selon revendication 1 d'antidotes de formule I pour la protection contre les dommages provoqués par des dérivés herbicides d'acides aryloxyphénoxypropioniques de formule IV

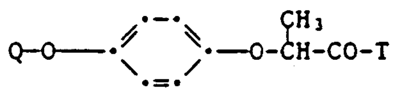

IV

dans laquelle
Q représente l'un des groupes

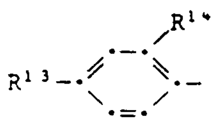 , 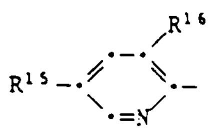 , 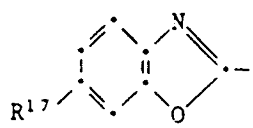 ,

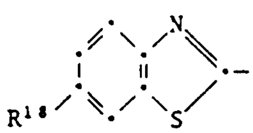 , ou 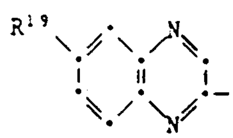

et
T représente -NR20R21, -N(CN)R22, -OR23, SR24 ou -O-N = CR25R26
R13 et R15 représentent des halogènes ou des groupes trifluorométhyle,
R14, R16, R17, R18 et R19 représentent l'hydrogène ou des halogènes,
R20 et R21 représentent chacun, indépendamment l'un de l'autre, l'hydrogène, un groupe alcoxy en C1-C8, alkyle en C1-C8, phényle ou benzyle, ou bien
R20 et R21 forment, ensemble et avec l'atome d'azote auquel ils sont reliés, un hétérocycle azoté saturé de 5 à 6 chaînons qui peut être interrompu par un atome d'oxygène ou de soufre,
R22 représente un groupe alkyle en C1-C4, alcényle en C3-C4, alcynyle en C3-C4 ou alcoxyalkyle en C2-C4,
R23 représente l'hydrogène ou un équivalent d'un ion de métal alcalin, de métal alcalinoterreux, de cuivre ou de fer ; un radical (alkyle en C1-C4)-ammonium ou (hydroxyalkyle en C1-C4)-ammonium quaternaire ; un groupe alkyle en C1-C9 portant éventuellement un ou plusieurs substituants choisis parmi les groupes amino, les halogènes, les groupes hydroxy, cyano, nitro, phényle, alcoxy en C1-C4, polyéthoxy contenant 2 à 6 motifs oxyde d'éthylène, -C0OR27, -COSR28, -CONH2, -CON-(alcoxy en C1-C4)-alkyle en C1-C4, -CO-N-di-(alkyle en C1-C4), CONH-alkyle en C1-C4, -N-(alcoxy en C1-C4)-alkyle en C1-C4 ou di-(alkyle en C1-C4)-amino ; un groupe alcényle en C3-C9 éventuellement substitué par des halogènes ou des groupes alcoxy en C1-C4, un groupe alcynyle en C3-C9 éventuellement substitué par des halogènes ou des groupes alcoxy en C1-C4 ; un groupe cycloalkyle en C3-C9 ; ou bien un groupe phényle éventuellement substitué par des groupes cyano, alkyle en C1-C4, alcoxy en C1-C4, acétylé, -COOR29, -COSR30, -CONH2, -CON-(alcoxy en C1-C4)-alkyle en C1-C4, -CO-N-di-(alkyle en C1-C4)- ou-CONH-alkyle en C1-C4,
R25 et R26 représentent chacun, indépendamment l'un de l'autre, un groupe alkyle en C1-C4 ou forment ensemble une chaîne alkylène de 3 à 6 chaînons et

R27, R28, R29 et R30 représentent chacun, indépendamment les uns des autres, l'hydrogène, un groupe alkyle en C1-C6, halogénoalkyle en C1-C6, alcoxyalkyle en C2-C6, alcényle en C3-C6, halogénoalkyle en C3-C6, alcynyle en C3-C6 ou halogénoalcynyle en C3-C6.

**9.** Utilisation selon revendication 1 d'antidotes de formule I pour la protection contre les dommages provoqués par des dérivés herbicides de N-benzoyl-N-phénylalanines de formule V

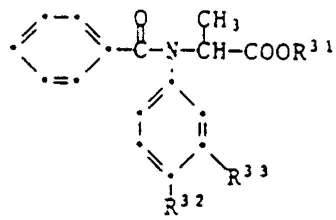

V

dans laquelle
R31 représente l'hydrogène ou un groupe alkyle en C1-C4 et
R32 et R33 représentent chacun, indépendamment l'un de l'autre, le fluor ou le chlore,

**10.** Procédé pour protéger les végétaux cultivés contre les dommages provoqués par des herbicides, caractérisé en ce que l'on traite la plante cultivée ou son habitat ou des semences ou pousses de la plante cultivée avant, durant ou après l'application de l'herbicide, par une quantité antagoniste efficace d'un composé de formule I

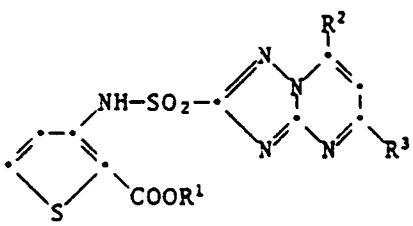

(I),

dans laquelle
R1 représente un groupe alkyle en C1-C6 ou cycloalkyle en C3-C6 et
R2 et R3 représentent chacun, indépendamment l'un de l'autre, l'hydrogène, un groupe alkyle en C1-C3, trifluorométhyle ou cyclopropyle,
sous réserve qu'un seul des symboles R2 et R3 peut représenter l'hydrogène.

**11.** Produit herbicide, caractérisé en ce qu'il contient un herbicide et une quantité suffisante pour protéger les végétaux cultivés contre les dommages provoqués par cet herbicide, d'un composé de formule I

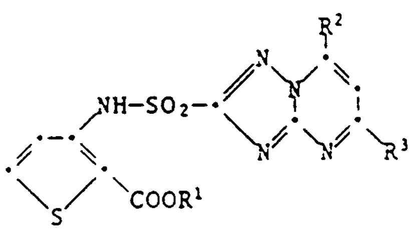

I

dans laquelle
R1 représente un groupe alkyle en C1-C6 ou cycloalkyle en C3-C6 et
R2 et R3 représentent chacun, indépendamment l'un de l'autre, l'hydrogène, un groupe alkyle en C1-C3, trifluorométhyle ou cyclopropyle,
sous réserve qu'un seul des symboles R2 et R3 peut représenter l'hydrogène, et le cas échéant des produits auxiliaires et/ou des véhicules.

**12.** Produit herbicide selon revendication 11, caractérisé en ce qu'il contient en tant qu'herbicide un dérivé de sulfonylurée de formule II selon la revendication 6.

**13.** Produit herbicide selon revendication 11, caractérisé en ce qu'il contient en tant qu'herbicide un chloracétanilide de formule III selon la revendication 7.

**14.** Produit herbicide selon revendication 11, caractérisé en ce qu'il contient en tant qu'herbicide un dérivé d'acide aryloxyphénoxypropionique de formule IV selon la revendication 8.

**15.** Produit herbicide selon revendication 11, caractérisé en ce qu'il contient en tant qu'herbicide une N-benzoyl-N-phénylalanine de formule V selon la revendication 9,

**16.** Produit servant à protéger les végétaux cultivés contre les dommages provoqués par les herbicides, caractérisé en ce qu'il consiste en au moins un composé de formule I'

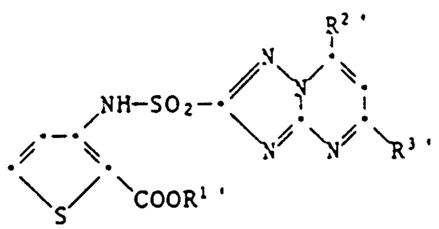

I'

dans laquelle
R1' représente un groupe alkyle en C1-C6 ou cycloalkyle en C3-C6 et
R2' et R3' représentent chacun, indépendamment l'un de l'autre, l'hydrogène, un groupe alkyle en C1-C3, trifluorométhyle ou cyclopropyle,
sous réserve que l'un au moins des symboles R2 et R3 représente un groupe cyclopropyle et un ou plusieurs produits auxiliaires et/ou véhicules,

**17.** Composés de formule I'

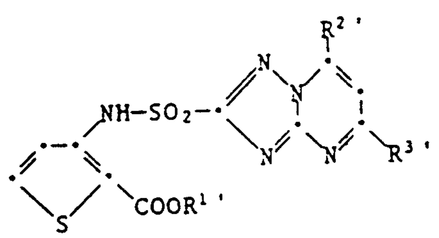

I'

dans laquelle
R1' représente un groupe alkyle en C1-C6 ou cycloalkyle en C3-C6 et
R2' et R3' représentent chacun, indépendamment l'un de l'autre, l'hydrogène, un groupe alkyle en C1-C3, trifluoromethyle ou cyclopropyle,
sous réserve que l'un au moins des symboles R2 et R3 représente un groupe cyclopropyle.

**18.** Composés de formule I' selon revendication 17, caractérisés en ce que l'un des symboles R2' et R3' représentent un groupe cyclopropyle et l'autre un groupe méthyle, R1' ayant les significations indiquées en référence à la formule I'.

**19.** Composés de formule I' selon la revendication 17, caractérisés en ce que R1' représente un groupe méthyle et R2' et R3' ont les significations indiquées en référence à la formule I'.

**20.** Composés de formule I' selon la revendication 17, caractérisés en ce que R1 représente un groupe méthyle et l'un des symboles R et R3 représente un groupe cyclopropyle et l'autre un groupe méthyle.

**21.** Composés de formule I' selon la revendication 17, caractérisés en ce que R1' représente un groupe alkyle en C1-C3 et R2' et R3' représentent chacun, indépendamment l'un de l'autre, l'hydrogène, un groupe méthyle ou cyclopropyle, sous réserve qu'un seul des symboles R2 et R3 peut représenter l'hydrogène.

**22.** Procédé de préparation des composés de formule I'

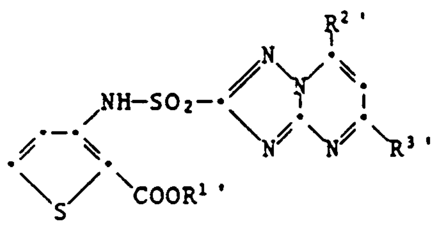

I'

dans laquelle
R1' représente un groupe alkyle en C1-C6 ou cycloalkyle en C3-C6 et
R2' et R3' représentent chacun, indépendamment l'un de l'autre, l'hydrogène, un groupe alkyle en C1-C3, trifluorométhyle ou cyclopropyle,
sous réserve que l'un au moins des symboles R2 et R3 représente un groupe cyclopropyle,
caractérisé en ce que l'on fait réagir un composé de formule VI

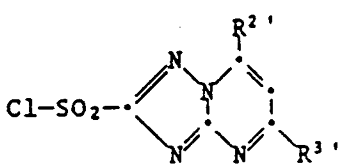

VI

dans laquelle
R2' et R3' représentent chacun, indépendamment l'un de l'autre, l'hydrogène, un groupe alkyle en C1-C3, trifluorométhyle ou cyclopropyle,
sous réserve que l'un au moins des symboles R2' et R3' représente un groupe cyclopropyle, avec un composé de formule VII

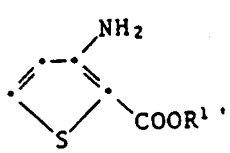

VII

dans laquelle R1' représente un groupe alkyle en C1-C6 ou cycloalkyle en C3-C6.

**23.** Procédé de préparation des composés de formule VI

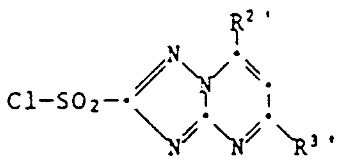

VI

dans laquelle
R2' et R3' représentent chacun, indépendamment l'un de l'autre, l'hydrogène, un groupe alkyle en C1-C3, trifluorométhyle ou cyclopropyle,
sous réserve que l'un au moins des symboles R2' et R3' représentent un groupe cyclopropyle, caractérisé en ce que l'on traite un composé de formule VIII

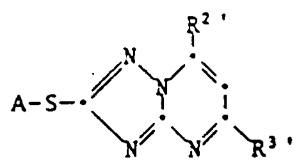

VIII

dans laquelle
R2' et R3' représentent chacun, indépendamment l'un de l'autre, l'hydrogène, un groupe alkyle en C1-C3, trifluorométhyle ou cyclopropyle,
sous réserve que l'un au moins des symboles R2' et R3' représente un groupe cyclopropyle, et
A représente l'hydrogène ou un groupe benzyle, par le chlore en milieu aqueux acide,

**24.** Procédé de préparation des composés de formule VIII

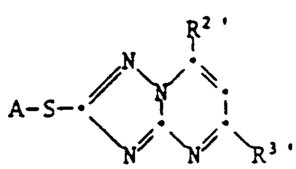

VIII

dans laquelle
R2' et R3' représentent chacun, indépendamment l'un de l'autre, l'hydrogène, un groupe alkyle en C1-C3, trifluorométhyle ou cyclopropyle,
sous réserve que l'un au moins des symboles R2' et R3' représente un groupe cyclopropyle, et
A représente l'hydrogène ou un groupe benzyle, caractérisé en ce que l'on fait réagir un composé de formule IX

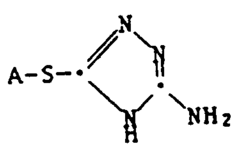

IX

dans laquelle
A représente l'hydrogène ou un groupe benzyle, avec un composé de formule X

$R_b$-CO-$CH_2$-$R_a$ X

dans laquelle

i) Ra représente le groupe -C0-$R_c$ et l'un des symboles $R_b$ et $R_c$ représente R2' et l'autre R3', ou bien

ii) Ra représente le groupe -CH(ORd)2 et Rb représente R ou R et Rd représente un groupe méthyle ou éthyle.

**25.** Composés de formule VI

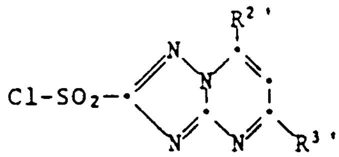

VI

dans laquelle R2 et R3 représentent chacun, indépendamment l'un de l'autre, l'hydrogène, un groupe alkyle en C1-C3, trifluorométhyle ou cyclopropyle, sous réserve que l'un au moins des symboles R et R3 représente un groupe cyclopropyle.

**26.** Composés de formule VIII

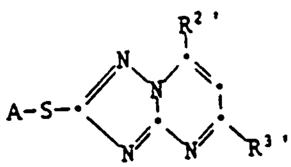

VIII

dans laquelle R2 et R3 représentent chacun, indépendamment l'un de l'autre, l'hydrogène, un groupe alkyle en C1-C3, trifluorométhyle ou cyclopropyle, sous réserve que l'un au moins des symboles R2 et R3 représente un groupe cyclopropyle, et A représente l'hydrogène ou un groupe benzyle.

**27.** Semences de végétaux cultivés qui ont été traitées par une quantité antagoniste efficace d'un composé de formule I.

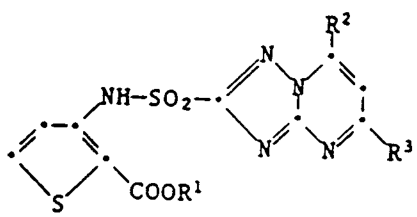

I

dans laquelle R1 représente un groupe alkyle en C1-C6 ou cycloalkyle en C3-C6, R représentent chacun, indépendamment l'un de l'autre, l'hydrogène, un groupe alkyle en C1-C3, trifluorométhyle ou cyclopropyle, sous réserve qu'un seul des symboles R et R peut représenter l'hydrogène.

**28.** Procédé pour combattre sélectivement les végétaux adventices dans les cultures de végétaux utiles, caractérisé en ce que l'on traite la culture, des parties de la plante cultivée ou l'aire de culture par un herbicide et un composé de formule I

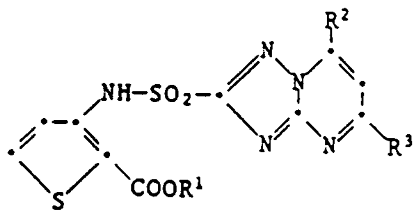

I

dans laquelle R représente un groupe alkyle en C1-C6 ou cycloalkyle en C3-C6 et R et R3 représentent chacun, indépendamment l'un de l'autre, l'hydrogène, un groupe alkyle en C1-C3, trifluorométhyle ou cyclopropyle, sous réserve qu'un seul des symboles R2 et R peut représenter l'hydrogène.